# EUROPEAN PATENT APPLICATION

(11) **EP 4 461 198 A1**
(43) Date of publication of application: **13.11.2024**
(21) Application number: 23887526.4
(22) Date of filing: 18.07.2023
(51) Int. Cl.: A61B 1/00, A61B 1/005

(54) **LOCKING APPARATUS FOR ENDOSCOPE, ENDOSCOPE, AND LOCKING APPARATUS**

(30) Priority: 08.11.2022 CN 202211394554
(71) Applicant: Micro-Tech (Nanjing) Co., Ltd., Nanjing, Jiangsu 210032 (CN)
(72) Inventor: JIN, Hongyan, Nanjing, Jiangsu 210032 (CN); SHI, Qiangqiang, Nanjing, Jiangsu 210032 (CN)
(74) Representative: Ullrich & Naumann PartG mbB
(86) International application number: PCT/CN2023/107953
(87) International publication number: WO 2024/098830

(57) **Abstract**

The present disclosure provides a locking device for an endoscope, an endoscope and a locking device. The locking device includes a first rotating wheel, a first rotating shaft, a brake disc, and a brake disc driving assembly; wherein the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; wherein a first traction wire may be adjusted via the rotation of the first traction disk, thereby adjusting a viewing angle of the endoscope in a first dimension; and the brake disc is arranged on at least one side of an outer circumferential surface of the first rotating shaft; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range. The locking device for an endoscope provided by this disclosure may improve the flexibility and stableness in set-up of a lens angle during an operation of the endoscope.

## Description

### FIELD OF THE INVENTION

The present disclosure pertains to the field of medical instruments, and in particular is related to a locking device for an endoscope, an endoscope, and a locking device.

### BACKGROUND OF THE INVENTION

With the reform of the national medical system, the advancement of medical technology, and the popularization of advanced medical equipment, endoscopic equipment is more and more widely used in routine medical surgeries.

As a commonly used medical instrument, endoscope equipment mainly consists of traction wires, bending parts, light sources, and lenses etc. In order to facilitate an observation of a lesion site, a locking device will be installed at a rear end of the endoscope. During actual applications, lens at a front end of the endoscope enters the human body through a minimally invasive incision. Usually, the lens at the front end of the endoscope will be adjusted to an appropriate position, and then the lens of the endoscope will fixed at a certain angle. A movement of the bending part is controlled by a locking device provided at a rear end of the endoscope, by which an adjustment of a viewing angle of the lens at the front end of the endoscope is achieved, so as to directly observe the condition of lesions in relevant regions. Therefore, as an important component in practical surgical operations, an endoscope locking device plays a vital role in observation of the lesion site.

In prior endoscope locking devices, a locking handwheel is usually used to adjust and lock the lens. Due to problems such as insufficient stability, low positioning accuracy, and in-flexibility to bend encountered during manipulation of the locking handwheel for controlling the lens in operation, it has become a technical problem that those skilled in the art need to solve to provide a locking device for an endoscope which may improve the flexibility to bend and the stability of the endoscope lens during operation.

### SUMMARY OF THE INVENTION

To solve the problems of insufficient operation stability, low precision and in-flexibility in current endoscope locking devices, the present disclosure provides a locking device for an endoscope. Additionally, the present disclosure provides an endoscope using the above locking device and a locking device.

A locking device for an endoscope according to an embodiment of the present disclosure includes a first rotating wheel, a first rotating shaft, a brake disc, and a brake disc driving assembly; wherein the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; wherein a first traction wire may be adjusted via the rotation of the first traction disk, thereby adjusting a viewing angle of the endoscope in a first dimension; and the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range.

According to an aspect of the present disclosure, the brake disc as a whole is configured as a frame structure clamping on the circumferential surface of the first rotating shaft or the circumferential surface of the first traction disk, wherein the frame structure forms a brake disc opening that may be opened/closed in an up-down direction; the brake disc driving assembly has a fastener provided at least on one end of the brake disc opening, and a force applied to the brake disc may be adjusted by the fastener, such that the tightness of engagement between the brake disc and the outer circumferential surface of the first rotating shaft or the outer circumferential surface of the first traction disk may be adjusted by changing a clamping force exerted by the brake disc on the first rotating shaft or the first traction disk, so that the rotational damping on the first rotating shaft may fall within the first preset damping range.

According to an aspect of the present disclosure, a protrusion is provided at a base of the brake disc that is opposite to the brake disc opening, such that the brake disc may be fixed within a case of the locking device via the protrusion.

According to an aspect of the present disclosure, a gap is provided at the base of the brake disc to increase a space for elastic deformation of the brake disc.

According to an aspect of the present disclosure, the protrusion is arranged between a first clamping post and a second clamping post provided on the case, so that the brake disc may be fixed within the case of the locking device.

According to an aspect of the present disclosure, the fastener includes: a stud, and an upper nut and a lower nut that cooperate with the stud; the upper nut is arranged at an upper port of the brake disc opening, and the lower nut is arranged at a lower port of the brake disc opening; wherein the upper nut and a first thread segment of the stud form a first thread pair, and the lower nut and a second thread segment of the stud form a second thread pair, with the first thread pair having a thread direction opposite to that of the second thread pair; wherein the force applied to the brake disc may be adjusted by a rotation of the stud.

According to an aspect of the present disclosure, the fastener includes a stud, and an internal thread cooperating with the stud is arranged at the opening of the frame structure; wherein an upper port of the opening cooperates with a first segment of the stud to form a third thread pair, and a lower port of the opening cooperates with a second segment of the stud to form a fourth thread pair, with the third thread pair having a thread direction opposite to that of the fourth thread pair; wherein the force applied to the brake disc may be adjusted by a rotation of the stud.

According to an aspect of the present disclosure, the stud is provided with a gripping portion extending outside of a housing of the case, such that an operator of the locking device may use the gripping portion to rotate the stud.

According to an aspect of the present disclosure, the stud is provided with an end surface abutting against an outer surface of the housing of the case.

According to an aspect of the present disclosure, the locking device further includes a second rotating wheel and a second rotating shaft; wherein the second rotating wheel is coupled to a first end of the second rotating shaft; wherein the second rotating shaft is coaxially arranged with respect to first rotating shaft, and is sleeved onto the outer circumferential surface of the first rotating shaft; wherein a second traction disk is provided around the second rotating shaft at a position close to a second end of the second rotating shaft; wherein the second rotating shaft is controlled by the second rotating wheel to rotate, and in turn drives the second traction disk to rotate; wherein a second traction wire may be adjusted via the rotation of the second traction disk, thereby adjusting the viewing angle of the endoscope in a second dimension; the second dimension is a dimension for orientation that is different from the first dimension; wherein the brake disc as a whole also clamps on an outer circumferential surface of the second rotating shaft or outer circumferential surface of the second traction disk; when the brake disc exerts a clamping force on the first rotating shaft and the first traction disk, it also exerts a corresponding clamping force on the second rotating shaft and the second traction disk, such that a rotational damping on the second rotating shaft may fall within a second preset damping range.

According to an aspect of the present disclosure, the brake disc is arranged on one side of the outer circumferential surface of the first rotating shaft, and the tightness of engagement between the brake disc and the outer circumferential surface of the first rotating shaft may be adjusted via the brake disc driving assembly, so that the rotational damping on the first rotating shaft may fall within the first preset range.

According to an aspect of the present disclosure, the locking device for an endoscope further includes a second rotating wheel and a second rotating shaft; wherein the second rotating wheel is coupled to a first end of the second rotating shaft; wherein the second rotating shaft is coaxially arranged with respect to first rotating shaft; wherein a second traction disk is provided around the second rotating shaft at a position close to a second end of the second rotating shaft, and the brake disc is arranged on at least one side of an outer circumferential surface of the second rotating shaft; when the tightness of engagement between the brake disc and the outer circumferential surface of the first rotating shaft is adjusted, an engagement tightness between the brake disc and the outer circumferential surface of the second rotating shaft will be adjusted synchronously, so that a rotational damping on the second rotating shaft may fall within a second preset range; wherein a second traction wire of the endoscope may be adjusted via a rotation of the second traction disk, thereby adjusting the viewing angle of the endoscope in a second dimension; the second dimension is a dimension for orientation that is different from the first dimension.

According to an aspect of the present disclosure, an O-ring is sleeved onto the outer circumferential surface of the first rotating shaft and/or the outer circumferential surface of the second rotating shaft at a location where the brake disc fits on the first rotating shaft and/or the second rotating shaft.

According to an aspect of the present disclosure, the brake disc is provided with a surface texture for increasing friction at its fitting surface engaging with the outer circumferential surface of the first rotating shaft and/or the outer circumferential surface of the second rotating shaft.

According to an aspect of the present disclosure, a positioning hole is provided at one end of a brake disc body, and is rotatably sleeved on a fixing post of the housing of the locking device; the tightness of engagement between the brake disc and the first rotating shaft and the second rotating shaft may be adjusted by adjusting a swing angle of the brake disc about the fixing post.

According to an aspect of the present disclosure, an arc-shaped through hole is provided on the brake disc body of the brake disc; wherein the brake disc driving assembly includes a driving member, with a body of driving member being coaxially arranged with respect to the first rotating shaft; the driving member further has a cantilever that is connected to the body and extends radially towards one side; the cantilever is provided with a cantilever post that extends axially and is inserted into the arc-shaped through hole provided on the brake disc; wherein a rotation of the driving member will drive the cantilever post to slide within the arc-shaped through hole, which in turn will drive the brake disc to swing about the fixing post, thereby adjusting an angle by which the brake disc swings about the fixing post.

According to an aspect of the present disclosure, the brake disc driving assembly further includes a toggle lever having a length that is set to be significantly larger than a diameter of the body of the driving member; wherein one end of the toggle lever is fixedly connected to the body of the driving member, and the other end of the toggle lever extends outwardly in the radial direction and provides an easy-to-toggle operation surface; wherein a turning of the toggle lever will drive the driving member to rotate.

According to an aspect of the present disclosure, a spacer is provided at an axial gap formed between the first traction disk and the second traction disk.

According to an aspect of the present disclosure, the brake disc and the brake disc driving assembly are divided into two groups to provide damping for the first rotating shaft and the second rotating shaft, respectively.

An endoscope according to another embodiment of the present disclosure includes a first rotating wheel, a first rotating shaft, a brake disc, and a brake disc driving assembly; wherein the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; and the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range; wherein a first traction wire may be adjusted via the rotation of the first traction disk, thereby adjusting a viewing angle of the endoscope in a first dimension; with such an adjustment and the damping provided by the brake disc, the lens of the endoscope can be driven to rotate into a required viewing angle in the first dimension and stay there as needed.

A locking device according to a further embodiment of the present disclosure includes a first rotating wheel, a first rotating shaft, a brake disc, and a brake disc driving assembly; wherein the first rotating wheel is coupled to a first end of the first rotating shaft; a first functional disc is provided around the first rotating shaft at a position close to a second end of the first rotating shaft; wherein the first rotating shaft is controlled by the first rotating wheel to rotate, and in turn drives the first functional disc to rotate; and the brake disc is arranged on at least one side of an outer circumferential surface of the first rotating shaft or an outer circumferential surface of the first functional disc; a tightness of engagement between the brake disc and the outer circumferential surface of the first rotating shaft or the outer circumferential surface of the first functional disc may be adjusted by the brake disc driving assembly, such that a rotational damping on the first rotating shaft may fall within a first preset damping range.

According to the present disclosure, the locking device for an endoscope includes a first rotating wheel, a first rotating shaft, a brake disc, and a brake disc driving assembly; wherein the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; wherein a first traction wire may be adjusted via the rotation of the first traction disk, thereby adjusting a viewing angle of the endoscope in a first dimension; and the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a desired rotational damping may be provided onto the first rotating shaft. By this, during an operation of the locking device, via the traction of the first traction wire, the lens mounted on the bending part at the distal end of the endoscope may be reliably fixed after it has rotated to a satisfactory angle, thus achieving a flexible and stable set-up of the angle of the endoscope lens during an operation of the endoscope, which in turn improves the surgery efficiency.

According to a first preferable aspect of the present disclosure, the brake disc as a whole has a frame configuration/structure clamping on the circumferential surface of the first rotating shaft or the first traction disk, and the frame structure forms a brake disc opening that may be opened in an up-down direction. The brake disc driving assembly has a fastener. The fastener is at least provided at the opening side of the brake disc opening. The fastener can adjust the force applied to the brake disc, thereby changing the clamping force exerted by the brake disc onto the first rotating shaft and the first traction disk. By this, a tightness of engagement between the brake disc and the outer circumferential surface of the first rotating shaft or the outer circumferential surface of the first traction disk may be adjusted, such that a desired rotational damping may be provided onto the first rotating shaft. According to a further preferred aspect of this disclosure, the fastener is configured to have a thread pair structure. The above preferred implementation may realize a continuous adjustment of the clamping force, and may be reliably maintained at any adjustment position, thus may provide an operating feel of various degrees as desired.

According to a second preferable aspect of the present disclosure, the brake disc is arranged on one side of the outer circumferential surface of the first rotating shaft. The tightness of engagement between the brake disc and the outer circumferential surface of the first rotating shaft may be adjusted by the brake disc driving assembly, so as to provide a desired rotational damping on the first rotating shaft. This preferred implementation is advantageous in that it is simple in structure and convenient to operate.

### BRIEF DESCRIPTION OF THE DRAWINGS

The above and other purposes, features and advantages of the embodiments of the present disclosure will be more easily understood from the following detailed description with reference to the accompanying drawings. In the drawings, a plurality of embodiments of the disclosure will be described in an exemplary and non-limiting manner, in which:
FIG. 1A is a schematic structural cross-sectional view of a locking device for an endoscope provided by a first embodiment of the present disclosure;
FIG. 1B is a structural schematic diagram of a brake disc driving assembly of the locking device in FIG. 1A;
FIG. 2A is a schematic structural diagram of the locking device in FIG. 1A from another viewing angle;
FIG. 2B is a structural schematic diagram of the brake disc of the locking device in FIG. 2A;
FIG. 3 is a left side view corresponding to the locking device in FIG. 2A;
FIG. 4 is a top view corresponding to the locking device in FIG. 2A;
FIG. 5 is a structural schematic diagram of a first traction disk of the locking device in FIG. 2A;
FIG. 6 is a structural schematic diagram illustrating another connection mode between an upper plate of the brake disc and a lower plate of the brake disc of the locking device in FIG. 2A;
FIG. 7 is a schematic diagram illustrating a state where the locking device in FIG. 2A is locked;
FIG. 8 is a schematic structural cross-sectional view of a locking device provided by a second embodiment;
FIG. 9 is a structural schematic diagram of a first functional disc of the locking device provided by the second embodiment;
FIG. 10 is a schematic diagram illustrating a state where the locking device provided by the second embodiment is locked;
FIG. 11 is a schematic structural cross-sectional view of a locking device for an endoscope provided by a third embodiment;
FIG. 12 is a schematic diagram illustrating a state where the locking device for an endoscope in FIG. 11 is unlocked;
FIG. 13 is a schematic diagram illustrating a state where the locking device for an endoscope in FIG. 11 is locked;
FIG. 14 are structural schematic diagrams of the brake disc in the locking device for an endoscope in FIG. 11; among which, a perspective view of an overall structure of the brake disc is represented on the left side, and a front view for one disk in the brake disc is represented on the right side;
FIG. 15 is a structural schematic diagram of a driving member in the locking device for an endoscope in FIG. 11;
FIG. 16 is a structural schematic diagram of a first traction disk in the locking device for the endoscope in FIG. 11;
FIG. 17 is a schematic diagram illustrating an overall structure of an endoscope provided by a fourth embodiment;
FIG. 18 is a schematic structural cross-sectional view of a locking device for an endoscope provided by a fifth embodiment.

### Reference numerals:

### Part of reference numerals in the first embodiment:

10-locking device;
100-rotating wheel assembly; 110-first rotating wheel assembly; 111-first rotating wheel; 113-first rotating shaft; 115-first traction disk; 1151-first traction disk groove; 1153-first traction wire; 1155-first traction hole; 1157-first traction disk center bore; 130-second rotating wheel assembly; 131-second rotating wheel; 133-second rotating shaft; 135-second traction disk; 1351-second traction disk groove; 150-spacer;
300-brake disc; 310-brake disc body (frame structure); 311-upper plate of the brake disc; 313-lower plate of the brake disc; 315-brake disc opening; 3151-upper port over the brake disc opening; 3153-lower port below the brake disc opening; 317-gap; 330-protrusion;
500-brake disc driving assembly; 510-fastener; 511-stud; 513-upper nut; 515-lower nut; 530-gripping portion.

### Part of reference numerals in the second embodiment:

115'-first functional disc; 135'-second functional disc; for the rest numerals, please refer to the reference numerals of the first embodiment.

### Part of reference numerals in the third embodiment:

210-locking device;
2100-rotating wheel assembly; 2110-first rotating wheel assembly; 2111-first rotating wheel; 2113-first rotating shaft; 2115-first traction disk; 21151-first traction disk groove; 21153-first traction wire; 21155-first traction hole; 21157-first traction disk center bore;
2130-second rotating wheel assembly; 2131-second rotating wheel; 2133-second rotating shaft; 2135-second traction disk; 21351-second traction disk groove;
2150-spacer; 2170-O-type sealing ring; 2170-1-first set of O-type sealing rings; 2170-2-second set of O-type sealing rings;
2300-brake disc; 2310-brake disc body; 2310-1-first brake disk body, 2310-2-second brake disk body; 2330-brake disc dent; 2350-arc-shaped through hole; 2370-positioning hole;
2500-brake disc driving assembly; 2510-driving member; 2511-driving member peripheral plane; 2513-hollow hole of the driving member; 2515-driving member body; 2517-cantilever; 2519-cantilever post; 2530-toggle lever;
2700-handle; 2710-fixing post.

### Part of reference numerals in the fourth embodiment:

2-endoscope; 210-locking device; 20-light source assembly; 30-traction wire; 40-suction tube; 50-flushing tube; 60-cable connector; 70-lens; 80-bending part; 90-catheter; 2530-toggle lever; 2700-handle.

### Part of reference numerals in the fifth embodiment:

210'-locking device; 2115'-first functional disc; 2135'-second functional disc; for the rest numerals, please refer to the reference numerals of the third embodiment.

### DETAILED DESCRIPTION OF THE EMBODIMENTS

The embodiments of the present disclosure will be described in detail below. Examples of the embodiments are shown in the accompanying drawings, in which identical or similar reference numerals are used throughout to represent the same or similar elements or elements with the same or similar functions. The embodiments described below with reference to drawings are exemplary only for a purpose to illustrate the present disclosure, and should not be construed as limiting the present disclosure.

In the description of the present disclosure, it should be understood that the terms "center", "longitudinal", "transverse", "length", "width", "thickness", "up", "down", "front", "back", "left", "right", "vertical", "horizontal", "top", "bottom", "inside", "outside", "clockwise", "counterclockwise", "axial", "radial", "circumferential" and the like are used to indicate orientations or positional relationships based on the orientations or positional relationships shown in the accompanying drawings, and are only used for a convenient and simplified description of the present disclosure, rather than indicating or implying that the device or element referenced must have a specific orientation, be constructed and operated in a specific orientation, and therefore cannot be understood as a limitation on the present disclosure.

In addition, the terms "first" and "second" are used for illustrative purpose only and should not be understood as indicating or implying relative importance or implicitly indicating the number of the indicated technical features. Therefore, features defined by "first" or "second" may explicitly or implicitly mean that the structure may comprise at least one of these features. In the description of this disclosure, the term "multiple" means two or more members, such as two members, three members, etc., unless otherwise clearly and specifically defined.

In this disclosure, unless otherwise clearly specified and defined, the terms "installed", "connected", "connection", "fix" and the like should be understood in a broad sense; for example, they may refer to a fixed connection, a detachable connection, or an integral connection. They may refer to a direct connection or an indirect connection via an intermediate media, or may mean an internal communication between two elements or an interaction between two elements, unless otherwise expressly defined. For those ordinary skilled in the art, the specific meanings of the above terms in this disclosure can be understood according to specific circumstances.

In this disclosure, unless otherwise expressly stated and defined, a first feature being "on" or "below" a second feature may mean that the first and second features are in direct contact, or the first and second features are in indirect contact via an intermediate media. Moreover, the first feature is "on", "above" and "on top of' the second feature may mean that the first feature is directly above or diagonally above the second feature, or may simply mean that the first feature has a larger horizontal height than the second feature. The first feature is "under", "below" and "beneath" the second feature may mean that the first feature is directly below or diagonally below the second feature, or may simply mean that the first feature has a smaller horizontal height than the second feature.

In current conventional endoscopes, a locking handwheel is usually used to adjust the lens of the endoscope. During this operation, many problems may be encountered in manipulation of the lens by endoscope locking device, such as insufficient stability, low positioning accuracy, and in-flexibility to bend.

In view of this, the present disclosure provides a locking device for an endoscope that includes a first rotating wheel, a first rotating shaft, a brake disc, and a brake disc driving assembly; wherein the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; wherein a first traction wire may be adjusted via the rotation of the first traction disk, thereby adjusting a viewing angle of the endoscope in a first dimension; and the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range, and in turn the first rotating shaft may be provide with a desired rotational damping. By applying rotational damping onto the first rotating shaft as mentioned above, the first traction disc may control a retraction and release of the first traction wire, and realize an adjustment of the angle and position of the endoscope lens via the first traction wire. This is beneficial for a bending and fixing of the distal bending part of the endoscope at any angle, thus achieving a flexible and stable set-up of the angle of the endoscope lens during an operation of the endoscope, which in turn improves the surgery efficiency.

A couple of optional implementations of the present disclosure will be introduced below in conjunction with the accompanying drawings. Those skilled in the art should understand that the following implementations are only illustrative and not an exhaustive listing. Based on these implementations, those skilled in the art may replace, disassemble or combine certain features or examples, which should still be regarded as falling within the content of the present disclosure.

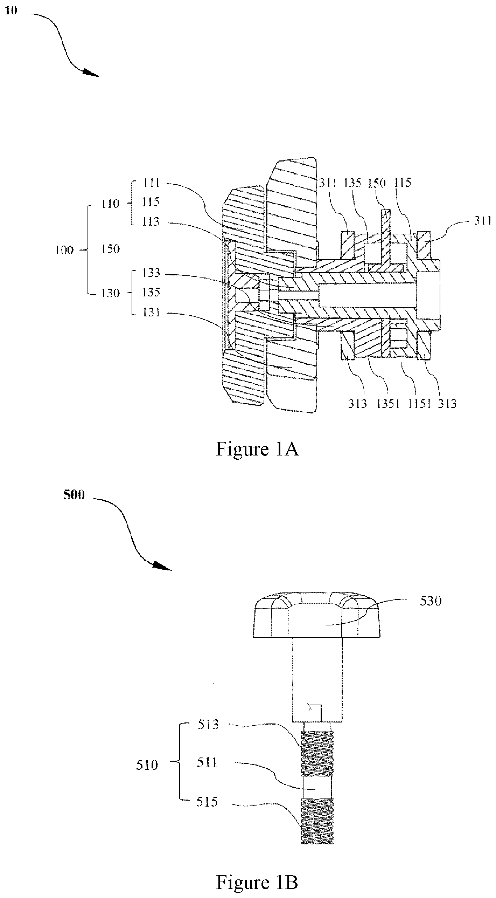

The first embodiment of the present disclosure will be introduced in detail below in conjunction with FIGS. 1A to 7. This embodiment is advantageous in that it may provide a continuous and stable feel during operation and may have a high adjustment reliability.

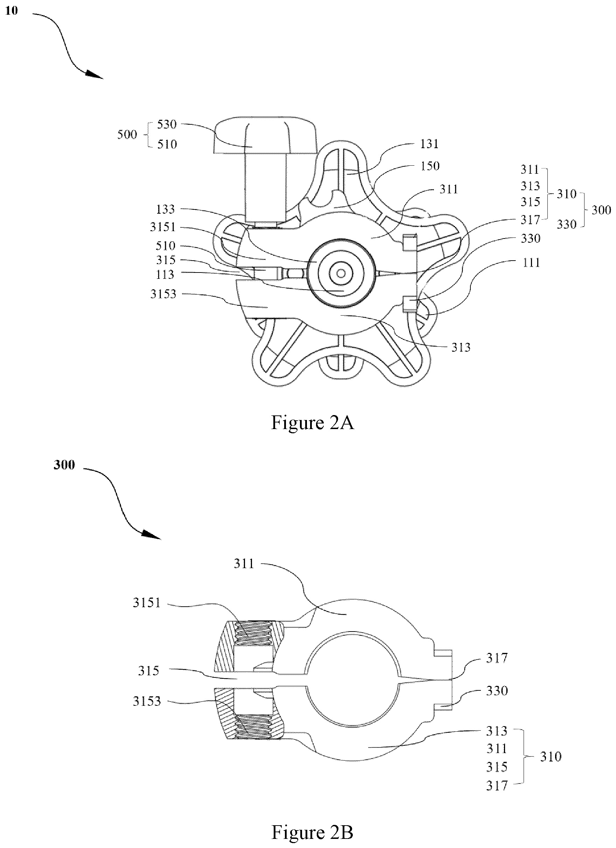

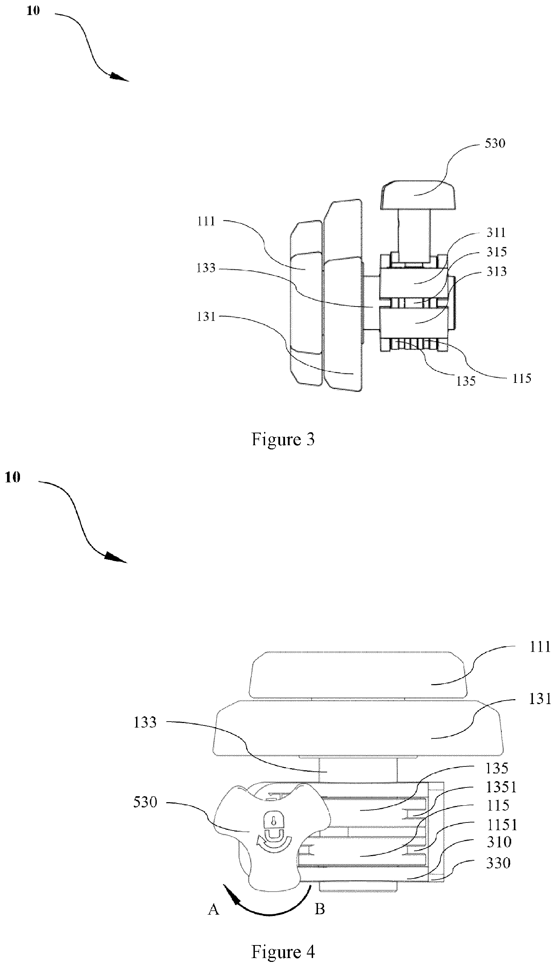

As shown in FIG. 1A, a schematic structural cross-sectional diagram of the locking device 10 for an endoscope is provided in this embodiment. In this figure, the left side in FIG. 1A is the first end of the locking device 10, i.e., the end where the rotating wheel assembly 100 is provided; the right side in FIG. 1A is the second end of the locking device 10, i.e., the end where the brake disc 300 is provided. In this figure, due to its viewing angle, the brake disc driving assembly 500 cannot be showed; the brake disc driving assembly 500 can be understood by referring to FIG. 1B and subsequent schematic diagrams. FIG. 1B shows the structure of the brake disc driving assembly 500. FIG. 2A is a structural schematic diagram of the locking device 10 in FIG. 1A from another viewing angle, i.e., a structural schematic diagram observed from the end where the brake disc 300 is provided. In this figure, the locking device 10 is in an unlocked state. FIG. 3 is a left side structural schematic diagram of the locking device 10 in FIG. 2A. FIG. 4 is a top structural schematic diagram of the locking device 10 in FIG. 2A.

In combination with the schematic diagrams in FIG. 1A and 1B, it may be seen that the locking device 10 comprises the following assemblies or components: a rotating wheel assembly 100; a brake disc 300; and a brake disc driving assembly 500.

An arrangement of the above-mentioned components can be roughly described as follows. The rotating wheel assembly 100 is located at a first end of the locking device 10 (the left side in FIG. 1A), the brake disc 300 is located at a second end of the locking device 10 (the right side in FIG. 1A), and the brake disc driving assembly 500 is also located at the second end of the locking device 10 (the right side in FIG. 1A) and is arranged at one end of the brake disc 300. The brake disc driving assembly 500 is connected to the brake disc 300 through a fastener 510, so as to be able to apply a force to the brake disc 300.

According to this embodiment, the locking device 10 further comprises the following components: a handle (this component is not shown in the drawings of this disclosure, but as a necessary component during an operation of the endoscope, those skilled in the art can easily understand the purpose of this component). The handle is actually the case of the locking device 10, and provides a positioning foundation for other components. The reason to call it as a "handle" is because it functions as a handle in the overall structure of the endoscope, and it is generally designed as two covers engaged with each other that may be disassembled according to assembly/repair needs. In this embodiment, the handle has a hollow cylindrical shell made of plastic material, which is configured to provide a mounting region for the rotating wheel assembly 100 and the brake disc 300. In this embodiment, the above-mentioned case is coupled to the locking device 10 mainly via the following components: a first clamping post and a second clamping post. The brake disc 300 is fixed within the case of the locking device 10 via the first clamping post and the second clamping post.

Next, the rotating wheel assembly 100 will be introduced. The rotating wheel assembly 100 comprises: a first rotating wheel assembly 110, a second rotating wheel assembly 130, and a spacer 150.

Each of the components will be described in detail below.

The rotating wheel assembly 100 comprises: a first rotating wheel assembly 110, a second rotating wheel assembly 130, and a spacer 150. The first rotating wheel assembly 110 comprises: a first rotating wheel 111, a first rotating shaft 113, and a first traction disk 115. The second rotating wheel assembly 130 comprises: a second rotating wheel 131, a second rotating shaft 133, and a second traction disk 135.

The first rotating wheel 111 is coupled to a first end of the first rotating shaft 113, and serves as an operating handle provided on the first rotating shaft 113. In this embodiment, the first rotating wheel 111 is provided at the first end of the first rotating shaft 113 (left side in FIG. 1A). The first traction disk 115 is provided at the other end of the first rotating shaft 113, that is, the second end of the first rotating shaft 113 (right side in FIG. 1A). The second rotating wheel 131 is coupled to a first end of the second rotating shaft 133, and the second rotating shaft 133 and the first rotating shaft 113 are coaxial. In this embodiment, as one of the most possible arrangements, specifically, the above coaxial relationship is achieved by sleeving the second rotating shaft 133 on an outer circumferential surface of the first rotating shaft 113. The second traction disk 135 is arranged around the second rotating shaft 133 close to the second end. It can be seen from the drawing that the second traction disk 135 is arranged closer to the second end (right side in FIG. 1A) with respect to the first traction disk 115. In addition, the first rotating wheel 111 is provided closer to the first end with respect to the second rotating wheel 131, and a protrusion extending toward the second end is provided on the first rotating wheel 111. Meanwhile, a corresponding groove is provided in the second rotating wheel 131 so that the protrusion is received within the groove, thereby reducing an installation dimension of the first rotating wheel 111 and the second rotating wheel 131 in the axial direction. Of course, the coaxial arrangement of the first rotating shaft 113 and the second rotating shaft 133 can also be realized in other different ways. For example, the first rotating shaft 113 and the second rotating shaft 133 may be arranged with two respective ends facing each other. If such an arrangement is adopted, the layout of the entire locking mechanism will be obviously different from that of this embodiment, but their principles are not essentially different.

After introducing the structures and connection relationships for each of the above-mentioned rotating shafts and rotating wheels, the structures of the traction disks will be introduced next. Since the second traction disk 135 has the same structure as the first traction disk 115, for the structure of the second traction disk 135, one may refer to the description of the first traction disk 115, which will not be repeated herein.

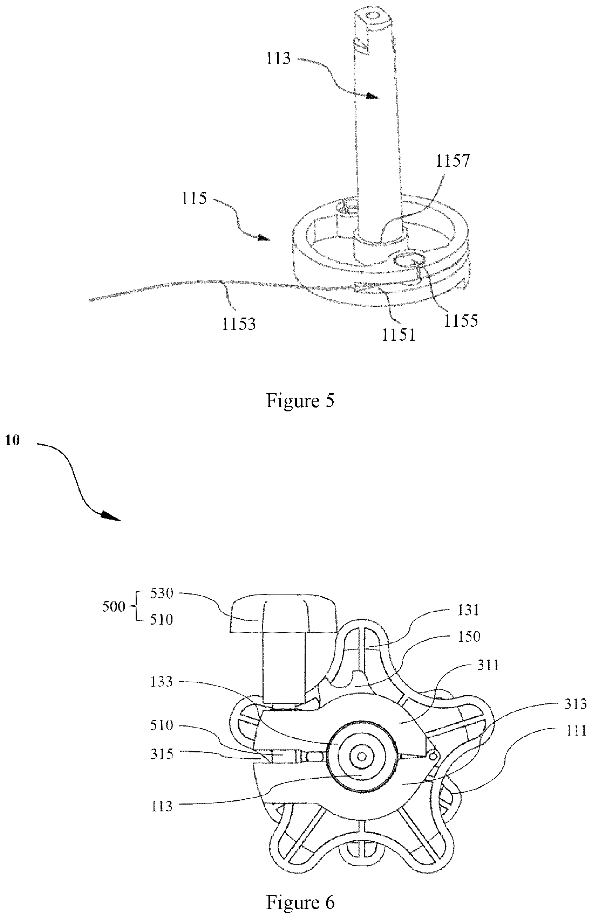

Referring to FIG. 5, which is a structural schematic diagram of the first traction disk 115 in the locking device 10. This figure also shows the first rotating shaft 113 connected to the first traction disk 115. The specific structure of the first traction disk 115 will be described in detail below in conjunction with FIG. 5, by referring to FIG. 1A also at the same time.

The first traction disk 115 is arranged at the second end of the first rotating shaft 113. The first traction disk 115 has thereon a position for fixing the first traction wire 1153. The first rotating shaft 113 may rotate under the control of the first rotating wheel 111, and drives the first traction disk 115 to rotate. An extension distance of the first traction wire 1153 may be adjusted via the rotation of the first traction disk 115; the first traction wire 1153 in turn will pull the lens of the endoscope, thereby adjusting a viewing angle of the endoscope in a first dimension and position the endoscope at a suitable angle.

As shown in FIG. 5, the first traction disk 115 comprises: a first traction disk groove 1151, a first traction wire 1153, a first traction hole 1155, and a first traction disk center bore 1157.

The first traction disk 115 has a hollow disk structure. The first traction disk groove 1151 is provided on an outer circumferential surface of the first traction disk 115. The first traction wire 1153 enters into the first traction disk 115 through the first traction disk groove 1151. Two bi-directional first traction holes 1155 are symmetrically provided along the circumferential surface of the first traction disk 115. The symmetrical first traction holes 1155 are configured for retraction and release of the first traction wire 1153. The first traction disk 115 is provided with a first traction disk center bore 1157 at the center of the disk, with the first rotating shaft 113 being nested and fixed therein. Via this structure, the first traction disk 115 is installed at the second end of the first rotating shaft 113.

One end of the first traction wire 1153 is fixed on the first traction disk 115 and can merge into the first traction disk groove 1151. The retraction and release of the first traction wire 1153 can be adjusted by rotating the first traction disk 115, thereby adjusting the viewing angle of the endoscope in the first dimension. Specifically, a rotation of the first traction disk 115 will control a winding/retraction or release of the first traction wire 1153 for the first dimension, thereby adjusting the extension distance of the first traction wire 1153. As a possible arrangement, in this embodiment, the first traction wire 1153 consists of two traction cables, and the viewing angle in the first dimension is in the up-down direction. The two traction cables of the first traction wire 1153 control the viewing angle of the endoscope in the up direction and down direction, respectively. In this embodiment, the configuration and control dimension of the above-mentioned first traction wire 1153 are merely illustrative, and it is not intended to exclude other possible configuration and control modes of the traction wire.

Similar to the first traction disk 115, the second traction disk 135 has a similar structure. The difference is that the second traction disk 135 is arranged around the second rotating shaft 133 at a position close to the second end. The second traction disk 135 is configured to fix the second traction wire, with the second traction wire being configured in a way similar to the first traction wire 1153 as mentioned above. A retraction and release of the second traction wire can be adjusted by rotating the second traction disk 135, thereby adjusting the viewing angle of the endoscope in a second dimension, e.g., the viewing angle of the endoscope in the left-right directions. Specifically, a rotation of the second traction disk 135 will control a winding/retraction or release of the second traction wire for the second dimension, thereby adjusting the extension distance of the second traction wire.

In this embodiment, the above-mentioned first traction wire 1153 and the second traction wire are both received within the catheter of the endoscope. For each traction wire, the two ends thereof are respectively positioned at the bending end and the traction disk end of the endoscope catheter. One end is connected to the corresponding traction disk, and the other end is fixed in the catheter. Generally speaking, in case one end of a traction wire is pulled by the corresponding traction disk, when the traction disk rotates, due to the flexibility of the catheter, the bending part at the distal end of the catheter will be driven by the traction wire to bend/flex, which in turn will drive the lens end of the endoscope to flex/rotate in a certain dimension (first, or second dimension), allowing the lens of the endoscope to deflect to a certain angle and thus changing the viewing angle.

In the above content, the rotating wheel assembly 100 has been introduced. The brake disc 300 will be introduced next.

Referring to the schematic diagrams of FIG. 2A and FIG. 2. FIG. 2A is a structural schematic diagram of the locking device 10 in an unlocked state. FIG. 2B is a schematic structural diagram of the brake disc 300.

The brake disc 300 comprises: a brake disc body 310 and a protrusion 330.

The brake disc body 310 is a frame structure clamping on a circumferential surface of the first rotating shaft 113 or the first traction disk 115. The frame structure is provided with a brake disc opening that can be opened/closed in an up-down direction. A clamping of the circumferential surface of the first rotating shaft 113 or the first traction disk 115 may be realized via this opening. A protrusion 330 is provided at a base of the frame structure of the brake disc body 310. The brake disc body 310 is fixed within the case of the locking device 10 via the protrusion 330. In this embodiment, specifically, the protrusion is arranged between the first clamping post and the second clamping post in the case, so that the brake disc body 310 is fixed within the case of the locking device 10.

The brake disc body 310 comprises: an upper plate 311 of the brake disc, a lower plate 313 of the brake disc, a brake disc opening 315, and a gap 317.

Referring to FIGS. 2A, 2B, 3 and 4, wherein FIG. 3 is a left side view corresponding to the locking device in FIG. 2A, and FIG. 4 is a top view corresponding to the locking device in FIG. 2A. The brake disc body 310 is a disc-shaped frame structure as a whole. The brake disc body 310 of the disc-shaped frame structure is composed of: an upper plate 311 of the brake disc which forms a semicircular frame, and a lower plate 313 of the brake disc which forms another semicircular frame. The upper plate 311 of the brake disc and the lower plate 313 of the brake disc are connected through the protrusion 330 provided at the base of the brake disc body 310, said protrusion being arranged opposite to the brake disc opening 315. The protrusion 330 provides a base positioning for the upper plate 311 of the brake disc and the lower plate 313 of the brake disc so that they may open and close with respect to each other. A gap 317 is provided in this region to increase a space for elastic deformation. The brake disc opening 315 is correspondingly formed between the above-mentioned upper plate 311 of the brake disc and the lower plate 313 of the brake disc. A clamping of the circumferential surface of the first rotating shaft 113 or the first traction disk 115 may be realized via the brake disc opening 315, so that a clamping force may be exerted on the first rotating shaft 113 and the first traction disk 115. At the opening side of the frame structure of the brake disc body 310, through holes are further provided, which are configured to connect with the brake disc driving assembly 500 and cooperate with it.

In this embodiment, as a specific implementation, a brake disc body 310 with a frame structure is specifically used, wherein the upper plate 311 of the brake disc and the lower plate 313 of the brake disc provided at one end of the frame structure are arranged on the outer circumferential surface of the first rotating shaft 113 (right side in FIG. 1A), and the upper plate 311 of the brake disc and the lower plate 313 of the brake disc at the other end of the frame structure are arranged on the outer circumferential surface of the second rotating shaft 133 (middle region in FIG. 1A). In addition, the upper plate 311 of the brake disc and the lower plate 313 of the brake disc are arranged opposite to each other on outer circumferential surfaces of respective rotating shaft; the upper plate 311 of the brake disc is arranged in an upper region on outer circumferential surfaces of respective rotating shaft (upper region of FIG. 2A), and the lower plate 313 of the brake disc is arranged in a lower region on outer circumferential surfaces of respective rotating shaft (lower region of FIG. 2A). A contact surface of the upper plate 311 of the brake disc and the lower plate 313 of the brake disc with each rotating shaft is provided with a surface texture for increasing friction. In addition, at the same side of the upper plate 311 of the brake disc and the lower plate 313 of the brake disc, i.e., at the opening side (left side in FIG. 2A), through holes are provided, and the brake disc driving assembly 500 is coupled with the brake disc body 310 via the through holes. In this embodiment, specifically the fastener of the brake disc driving assembly 500 is engaged/coupled via a through hole in an upper port 3151 of the brake disc opening and a through hole in a lower port 3153 of the brake disc opening.

As a possible way of this embodiment, as shown in FIG. 2A, the protrusion 330 is plate-shaped and is located on the same side of the upper plate 311 of the brake disc and the lower plate 313 of the brake disc, i.e., at the side opposite to the brake disc opening 315 (right side in FIG. 2A). The protrusion 330 opens toward the brake disc body 310 to hold/clamp on the upper plate 311 of the brake disc and the lower plate 313 of the brake disc from both sides, which provides positioning for the upper plate 311 of the brake disc and the lower plate 313 of the brake disc.

As another possible way of this embodiment, as shown in FIG. 6, which is a structural schematic diagram illustrating another connection mode between an upper plate 311 of the brake disc and a lower plate 313 of the brake disc of the locking device in FIG. 2A. As shown in FIG. 6, the upper plate 311 of the brake disc and the lower plate 313 of the brake disc are connected in a hinged manner, the protrusion 330 is of a shaft configuration, and the protrusion 330 of shaft configuration is inserted into a hinge/articulation site between the upper plate 311 of the brake disc and the lower plate 313 of the brake disc. The protrusion 330 of shaft configuration provides a rotation axis for the upper plate 311 of the brake disc and the lower plate 313 of the brake disc.

Next, the brake disc driving assembly 500 will be introduced. For ease of understanding, one could refer to the schematic diagram in FIG. 1B or 2A.

As shown in FIG. 1B, the brake disc driving assembly 500 comprises: a fastener 510; and a gripping portion 530.

The fastener 510 is arranged in the frame structure of the brake disc body 310 and is provided at the position of the brake disc opening 315. Specifically, the fastener 510 is arranged within the through holes in the brake disc body 310. The upper plate 311 of the brake disc and the lower plate 313 of the brake disc are connected through the fastener 510. The gripping portion 530 is provided at one end of the fastener 510, and extends out of the case of the locking device 10 so that an operator of the locking device 10 may reach and rotate the fastener 510. As a specific implementation, the gripping portion 530 is a knob. The knob has, in a radial direction perpendicular to the first rotating shaft 113, a radius that is set to be significantly larger than the radius of the first rotating wheel 111. A rotation of the knob will drive the fastener 510 to rotate, the fastener 510 in turn rotates within the through holes in the brake disc body 310, thus the force applied to the brake disc 300 may be adjusted. By this, the clamping force exerted onto the first rotating shaft 113 and the first traction disk 115 by the brake disc 300 may be adjusted, so that a rotational damping on the first rotating shaft 113 may fall within a first preset damping range; that is, a rotation of the first rotating shaft 113 is damped by a required degree. Among which, the first preset damping range is not specifically limited in the embodiments of this disclosure and can be adaptively adjusted according to the actual usage conditions of the locking device 10.

For ease of understanding, the fastener 510 will be introduced in detail next. The fastener 510 may be implemented in two ways, which will be introduced respectively below.

According to the first implementation, the fastener 510 comprises: a stud 511, an upper nut 513, and a lower nut 515.

As the main body of the fastener 510, the stud 511 is arranged to pass through the through holes in the brake disc body 310; At least two thread segments with opposite threading directions are provided on an outer circumferential surface of the stud 511. An upper nut 513 and a lower nut 515 are also provided to cooperation with the stud 511, wherein the upper nut 513 is fastened over the upper port 3151 of the brake disc opening, and the lower nut 515 is fastened beneath the lower port 3153 of the brake disc opening. The upper nut 513 and the first thread segment of the stud 511 form a first thread pair, and the lower nut 515 and the second thread segment of the stud 511 form a second thread pair. The first thread pair and the second thread pair have opposite thread directions. An adjustment of the force exerted on the brake disc 300 may achieved via a rotation of the stud 511. Since the thread directions of the first thread pair and the second thread pair are opposite to each other, a rotation of the stud 511 in one direction will cause the upper nut 513 and the lower nut 515 to move closer to each other along the axial direction of the stud 511, or vice versa. In this way, a clamping over the first rotating shaft 113 and the second rotating shaft 133 may be realized via the brake disc opening 315 of the brake disc body 310, thus achieving an increase or decrease of damping on the rotating shaft.

According to this embodiment, as another possible way, internal threads cooperating with (the threads on) the stud 511 are provided in the through holes at the opening of the frame structure of the brake disc body 310. Among which, a third thread pair is formed between the upper port 3151 of the brake disc opening and the cooperating first thread segment of the stud 511, and a fourth thread pair is formed between the lower port 3153 of the brake disc opening and the cooperating second thread segment of the stud 511. The third thread pair and the fourth thread pair have opposite thread directions. When the stud 511 rotates, threads provided on the surface of the stud 511 will cooperate with the upper port 3151 and the lower port 3153 of the brake disc opening, thereby applying force to the brake disc 300 or reducing the force; its working principle is similar to that of the previous implementation in which a nut is used.

According to the above two implementations, during operation of the endoscope, the gripping portion 530 is rotated to drive the stud 511 to rotate synchronously, so as to realize a cooperation between the stud 511 and the upper nut 513 and the lower nut 515 (the first implementation), or a cooperation between the stud 511 and the upper port 3151 and the lower port 3153 of the brake disc opening (the second implementation). Thus, an adjustment of the force exerted onto the brake disc body 310 may be achieved. Since the brake disc body 310 is of a frame structure with a brake disc opening 315 that can be opened in an up-down direction, the outer circumferential surface of the first rotating shaft 113 and the outer circumferential surface of the second rotating shaft 133 may be clamped by the brake disc body 310 mounted thereon. By adjusting the force applied to the brake disc body 310 in the above way, the clamping force applied by the brake disc body 310 onto the first rotating shaft 113 and the second rotating shaft 133 may be changed, so that the rotational damping on the first rotating shaft 113 may fall within the first preset damping range, and a rotational damping on the second rotating shaft 113 may fall within a second preset damping range. Thus, the required rotational damping may be provided for the first rotating shaft 113 and the second rotating shaft 133. Generally, the locking device 10 may have an unlocked state and a locked state, and may provide different degrees of damping in intermediate positions between these two states. Wherein, the second preset damping range is not specifically limited in this disclosure and can be adaptively adjusted according to the actual use conditions of the locking device 10. The first preset damping range may be the same as or different from the second preset damping range.

The working process of the locking device 10 will be described in detail below in conjunction with FIG. 2A, FIG. 4, and FIG. 7.

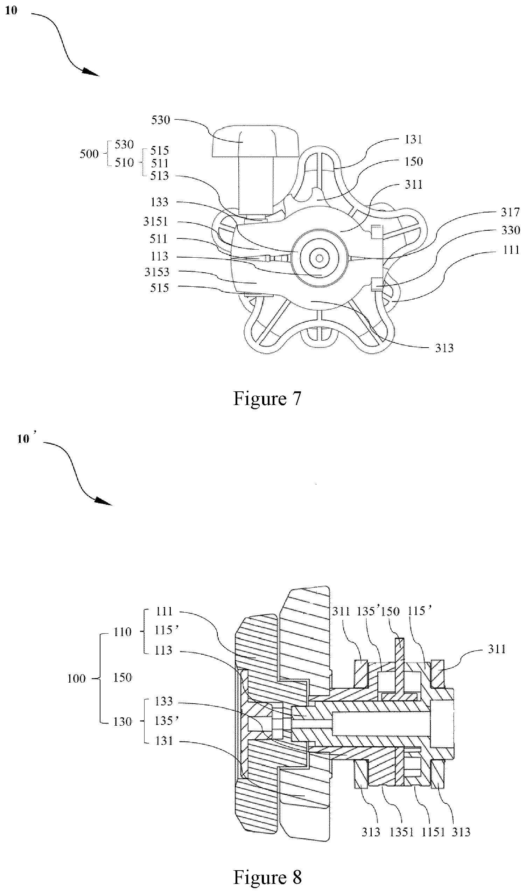

FIG. 2A shows a schematic diagram of the locking device 10 when it is in an unlocked state; FIG. 4 shows a process of switching the locking device 10 from the unlocked state to a locked state (FIG. 4 is a top view corresponding to the locking device in FIG. 2A); FIG. 7 shows a schematic diagram of the locking device 10 when it is in the locked state.

Both FIG. 2A and FIG. 7 are structural schematic diagrams of the locking device 10 in FIG. 1A, in which the locking device 10 is rotated clockwise so as to be observed from the end where the brake disc 300 is provided (i.e., from the second end of the locking device 10). The working process of the locking device 10 will be briefly described below with reference to FIG. 2A, FIG. 4, and FIG. 7, with emphasis on the switching process between the locked state and the unlocked state.

When it is required to lock the endoscope locking device 10, the knob of the gripping portion 530 will be rotated counterclockwise by a certain angle (from position B to position A as shown in FIG. 4). The knob of the gripping portion 530 drives the stud 511 to rotate counterclockwise. Since the first thread pair formed between the upper nut 513 and the first thread segment of the stud 511, and the second thread pair formed between the lower nut 515 and the second thread segment of the stud 511, with above-mentioned first thread pair and the second thread pair having opposite thread directions, the surface threads on the stud 511 will cooperate with the upper nut 513 and the lower nut 515 when the stud 511 rotates counterclockwise, causing the upper nut 513 and the lower nut 515 to move closer to each other along the axial direction of the stud 511. Since the upper nut 513 and the lower nut 515 are fastened on the inner surface of the through holes of the brake disc body 310, the opening degree of the brake disc opening 315 will gradually decrease, and the clamping force exerted by the brake disc body 310 of the frame structure on the first rotating shaft 113 and the second rotating shaft 133 will gradually increase. By this, the rotational damping on the first rotating shaft 113 and the second rotating shaft 133 may gradually increase. The first rotating shaft 213 and the second rotating shaft 233 gradually stop rotating under the action of the above-mentioned damping, and the first traction disk groove 2151 and the second traction disk groove 2351 in turn stop the retraction/release and winding of the traction wires that control a rotation of the lens in the left-right direction and up-down direction, respectively. Thus, for traction wires (designated for up-down traction and left-right traction, respectively), their traction length in the catheter will be fixed, and the locking device 10 enters into the locked state; that is, the angle and orientation of the endoscope lens will be locked. Referring to FIG. 2A and FIG. 7, the above process corresponds to a process changing from FIG. 2A to FIG. 7. It can be seen that when the upper plate 311 of the brake disc and the lower plate 313 of the brake disc are at the position of FIG. 2A, the brake disc opening 315 formed between them is larger, and when they are at the position of FIG. 7, the brake disc opening 315 formed between them is smaller.

When it is required to unlock the endoscope locking device 10, the knob of the gripping portion 530 will be rotated clockwise by a certain angle (from position A to position B as shown in FIG. 4). The knob of the gripping portion 530 drives the stud 511 to rotate clockwise. The surface threads on the stud 511 will cooperate with the upper nut 513 and the lower nut 515, causing the upper nut 513 and the lower nut 515 to move away from each other along the axial direction of the stud 511, and the opening degree of the brake disc opening 315 will gradually increase, and the clamping force exerted by the brake disc body 310 of the frame structure on the first rotating shaft 113 and the second rotating shaft 133 will gradually decrease. By this, the rotational damping on the first rotating shaft 113 and the second rotating shaft 133 may gradually decrease and disappear. The first rotating shaft 213 and the second rotating shaft 233 may rotate more easily when the damping decreases as mentioned above. When the above-mentioned damping disappears, the locking device 10 will be released from the above-mentioned locked state and enters into the above-mentioned unlocked state. At this time, the first traction disk 215 and the second traction disk 235 may rotate readily as the operator operates the first rotating wheel 211 and the second rotating wheel 231. Traction wires correlated with the above-mentioned traction disks can be readily wound and retracted/released under the traction of the traction disks, so that the angle of the endoscope lens may be flexibly adjusted. Referring to FIG. 2A and FIG. 7, the above process corresponds to a process changing from FIG. 7 to FIG. 2A. It can be seen that when the upper plate 311 of the brake disc and the lower plate 313 of the brake disc are at the position of FIG. 7, the brake disc opening 315 formed between them is smaller, and when they are at the position of FIG. 2A, the brake disc opening 315 formed between them is larger.

In a middle position between the above-mentioned locked position and unlocked position, the gripping portion 530 can be operated/set to different positions between A and B, so that the locking device 10 is under different damping, so as to adjust a tightness of the rotating wheels differently based on actual requirements. Through the locking device 10, the endoscope may be adjusted to a suitable state as needed. In the unlocked state, the lens angle of the endoscope can be freely adjusted via the rotating wheel. In the locked state, the endoscope is in a fixed state, and the lens angle will not change. In a damped state between the above two states, the present disclosure may provide the operator with the feel he needs to turn the wheel, thus making it easier to manipulate the device.

Similar to the first embodiment, as a preferred embodiment, it is obvious that there may be other variations with respect to its basic principle. For example, the rotating wheel assembly 100 may only be provided with the first rotating wheel assembly, that is, the locking device 10 can only adjust the angle of the endoscope in one dimension. Of course, there are some other possible variations. For example, the first rotating wheel 111 and the second rotating wheel 131 mentioned hereinabove may be oppositely arranged, rather than being arranged at the same end as in this embodiment.

Corresponding to the aforementioned first embodiment, the second embodiment of the present disclosure provides a locking device; the purpose of providing this embodiment is to extend the principle of the first embodiment to other possible disclosure scenarios, rather than being limited to endoscopes only.

Its structure and working process will be described below in conjunction with FIGS. 8-10. At the same time, reference can be made to FIG. 1A-FIG. 7 of the first embodiment. In this embodiment, elements with the same functions as those in the above-mentioned first embodiment are named similarly as much as possible to facilitate understanding. It should be noted that although there are common innovation points between the first embodiment and the second embodiment, there are still significant differences between these two embodiments. Therefore, the description of this embodiment will be based on the nomenclature provided in this embodiment, there is no need for them to correspond exactly to the first embodiment constrainedly.

The locking device 10' is usually used in detection scenarios. Its working process will be described in conjunction with the first embodiment. The first traction disk 115 and the second traction disk 135 in the rotating wheel assembly 100 are replaced with a first functional disc 115' and a second functional disc 135' which are used to realize various possible adjustment functions. It should be understood that the configuration of the first function disc 115' and the second function disc 135' can be structurally adjusted according to the functions and application occasions of the locking device 10'. This embodiment is not specifically limited.

The locking device 10' comprises: a first rotating wheel 111, a first rotating shaft 113, a brake disc 300, and a brake disc driving assembly 500.

The first rotating wheel 111 is coupled to a first end of the first rotating shaft 113. A first functional disc 115' is provided at a position of the first rotating shaft 113 close to its second end. The first rotating shaft 113 may be rotated under the control of the first rotating wheel 111, and in turn drives the first functional disc 115' to rotate. The first traction wire 1153 may be adjusted via a rotation of the first functional disc 115', thereby adjusting the viewing angle of the endoscope in the first dimension.

The brake disc 300 as a whole has a frame configuration/structure clamping on the circumferential surface of the first rotating shaft 113 or the first functional disc 115', and the frame structure forms a brake disc opening 315 that may be opened in up-down direction.

The brake disc driving assembly 500 has a fastener 510. The fastener 510 is arranged at the opening side of the brake disc opening 315. The fastener 510 can adjust the force applied to the brake disc 300, thereby changing the clamping force exerted by the brake disc 300 onto the first rotating shaft 113 and the first functional disc 115'. By this, a rotational damping on the first rotating shaft 113 may fall within a first preset damping range, that is, a required damping may be provided on the first rotating shaft 113 during its rotation.

The locking device 10' provided by the second embodiment described above may also comprise other necessary structures, such as a detection device and a display device used to cooperate with the locking device. It should be understood that the working process of the locking device of this embodiment is similar to that of the first embodiment. A reference is made to the first embodiment, and will not be described in detail in this embodiment. It should be emphasized that FIG. 8 to FIG. 10 appear to have the same structure as FIG. 1A, FIG. 2A and FIG. 5 of the first embodiment. They are only used to exemplarily show that the components in the second embodiment have the same functions as the components in the first embodiment, and do not intend to limit their structures to be the same. It should be understood that the components in the second embodiment that have the same functions as those in the first embodiment may be the same or different in structure.

The third embodiment of the present disclosure provides another locking device for an endoscope.

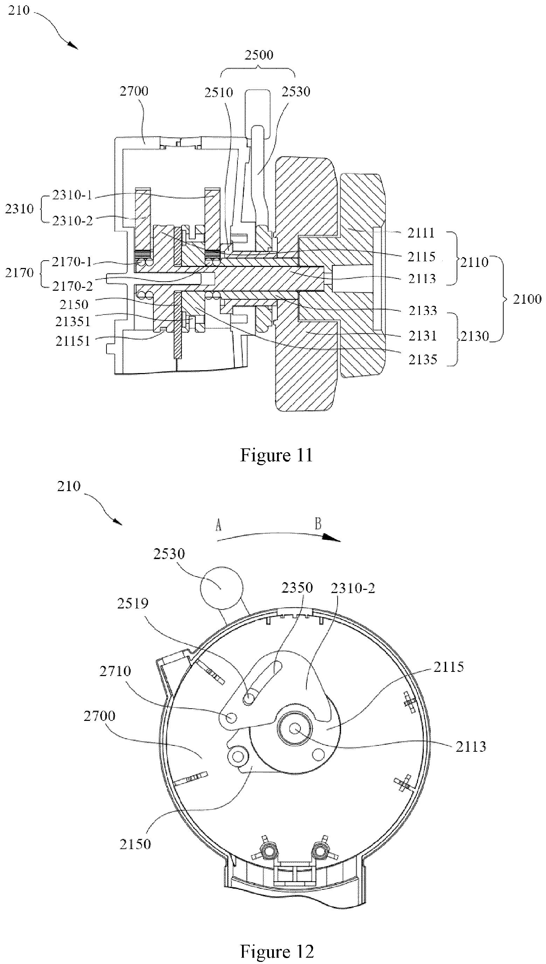

The third embodiment of the present disclosure will be introduced in detail below in conjunction with FIG. 11 to FIG. 16. This embodiment has essentially the same principle as the above-mentioned first and second embodiments, that is, the desired damping on the rotation of the rotating shaft is provided via an adjustment of the brake disc arranged on at least one side of the outer circumferential surface of the rotating shaft (the first rotating shaft and/or the second rotating shaft) or the traction disk (or the functional disc). However, in terms of the specific implementation of the brake disc, the third embodiment adopts a different structure, which has the advantage of being simpler in structure.

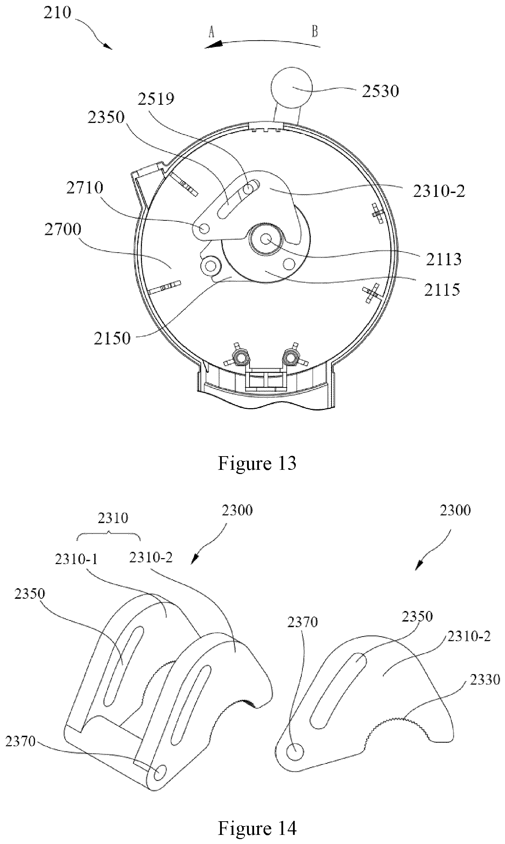

FIG. 11 illustrates a cross-sectional structural schematic diagram of the locking device 210 for the endoscope provided in this embodiment. Among which, the left side in FIG. 11 is the second end of the locking device 210, i.e., an end where the brake disc 2300 is provided; the right side in FIG. 11 is the first end of the locking device 210, i.e., an end where the rotating wheel assembly 2100 is provided. In this figure, the locking device 210 is in a locked state. FIG. 12 is a structural schematic diagram of the locking device 210 in FIG. 11 in an unlocked state. FIG. 13 is a structural schematic diagram of the locking device 210 in FIG. 11 in a locked state. Both FIG. 12 and FIG. 13 are structural schematic diagrams of the locking device 210 in FIG. 11, in which the locking device 210 is rotated counter-clockwise so as to be observed from the second end of the locking device 210, i.e., from the end on which the brake disc 2300 is provided.

As shown in FIG. 11, the locking device 210 comprises the following assemblies or components: a rotating wheel assembly 2100, a brake disc 2300, a brake disc driving assembly 2500, and a handle 2700.

The arrangement of the above-mentioned components can be roughly described as follows: the brake disc 2300 is arranged close to the second end of the locking device 210 (left side in FIG. 11), the brake disc driving assembly 2500 is provided approximately in a middle region of the locking device 210 (middle in FIG. 11), and the rotating wheel assembly 2100 is arranged at the first end of the locking device 210 (right side in FIG. 11). The brake disc 2300 and the brake disc driving assembly 2500 are coupled with each other via a driving member body 2510. The brake disc driving assembly 2500 provides rotational damping onto the rotating wheel assembly 2100 through the brake disc 2300.

In this embodiment, the handle 2700 is actually the case of the locking device 210 which provides a positioning foundation for other components. The reason to call it as a "handle" is because it functions as a handle in the overall structure of the endoscope, and its specific structure will be explained later.

The rotating wheel assembly 2100 comprises: a first rotating wheel assembly 2110, a second rotating wheel assembly 2130, an spacer 2150, and an O-ring seal 2170.

Each component will be described in detail below.

The rotating wheel assembly 2100 comprises: a first rotating wheel assembly 2110, a second rotating wheel assembly 2130, an spacer 2150 and an O-ring 2170. The first rotating wheel assembly 2110 comprises a first rotating wheel 2111, a first rotating shaft 2113, and a first traction disk 2115; and the second wheel assembly 2130 comprises a second wheel 2131, a second rotating shaft 2133, and a second traction disk 2135.

The first rotating wheel 2111 is connected to a first end of the first rotating shaft 2113, and serves as an operating handle provided on the first rotating shaft 2113. In this embodiment, the first rotating wheel 2111 is arranged at the first end of the first rotating shaft 2113 (right side in FIG. 11); the first traction disk 2115 is arranged at the other end of the first rotating shaft 2113, i.e., the second end of the first rotating shaft 2113 (left side in FIG. 11). The second rotating wheel 2131 is connected to the first end of the second rotating shaft 2133, and the second rotating shaft 2133 is coaxial with the first rotating shaft 2113. In this embodiment, as a most possible arrangement, specifically, a coaxiality is achieved by sleeving the second rotating shaft 2133 on the outer circumferential surface of the first rotating shaft 2113. The second traction disk 2135 is arranged around the second rotating shaft 2133 close to the second end. As can be seen from the drawing, the second traction disk 2135 is arranged closer to the first end (right side in FIG. 11) with respect to the first traction disk 2115. In addition, an O-ring 2170 is sleeved on the first rotating shaft 2130, and two groups of O-rings 2170 are provided. Each group has two O-rings that are arranged adjacent to each other; wherein the first group of O-rings 2170-1 is immediately close to the first traction disk 2115 and is arranged closer to the second end (the left side in FIG. 11) with respect to the first rotating shaft 2113, and the second group of O-rings 2170-2 is immediately close to the second traction disk 2135 and is arranged closer to the first end with respect to the second rotating shaft 2133 (the middle in FIG. 11). In addition, the first rotating wheel 2111 is provided closer to the first end with respect to the second rotating wheel 2131, and a protrusion extending toward the second end is provided on the first rotating wheel 2111. Meanwhile, a corresponding groove is provided in the second rotating wheel 2131, so that the protrusion is received within the groove, thereby reducing an installation dimension of the first rotating wheel 2111 and the second rotating wheel 2131 in the axial direction. Of course, the coaxial arrangement of the first rotating shaft 2113 and the second rotating shaft 2133 may also be realized in other different ways. For example, they may be arranged with two respective ends facing each other. If such an arrangement is adopted, the layout of the entire locking mechanism will be obviously different from that of this embodiment, but their principles are not essentially different.

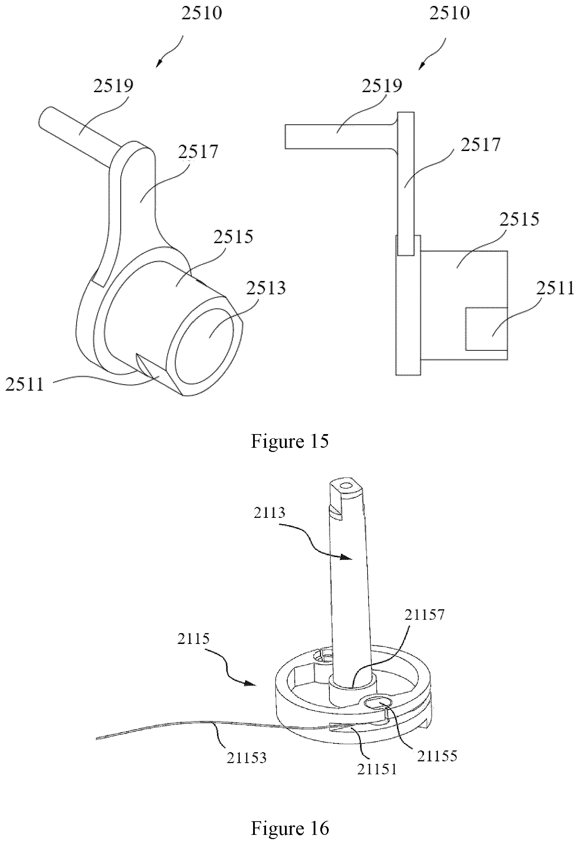

Referring to FIG. 16, the first traction disk 2115 has a position for fixing the first traction wire 21153. An extension distance of the first traction wire 21153 may be adjusted via the rotation of the first traction disk 2115; the first traction wire 21153 in turn will pull the lens of the endoscope, thereby adjusting the endoscope to a suitable viewing angle of in a dimension controlled by the first traction wire 21153.

FIG. 16 shows a structural diagram of the first traction disk 2115. This figure also shows a first rotating shaft 2113 connected to the first traction disk 2115. The specific structure of the first traction disk 2115 is described in detail below in conjunction with FIG. 16, and FIG. 11 may be referred to at the same time.

As shown in FIG. 16, the first traction disk 2115 comprises: a first traction disk groove 21151, a first traction wire 21153, a first traction hole 21155, and a first traction disk center bore 21157.

The first traction disk 2115 has a hollow disc structure. The first traction disk groove 21151 is provided on an outer circumferential surface of the first traction disk 2115. The first traction wire 21153 enters into the first traction disk 2115 through the first traction disk groove 21151. Two bi-directional first traction holes 21155 are symmetrically provided along the circumferential surface of the first traction disk 2115. The symmetrical first traction holes 21155 are configured for retraction and release of the first traction wire 21153. The first traction disk 2115 is provided with a first traction disk center bore 21157 at the center of the disk, with the first rotating shaft 2113 being nested and fixed therein. Through this structure, the first traction disk 2115 is installed at the second end of the first rotating shaft 2113.

One end of the first traction wire 21153 is fixed on the first traction disk 2115 and can be merge into the first traction disk groove 21151. The retraction and release of the first traction wire 21153 can be adjusted by rotating the first traction disk 2115, thereby adjusting the viewing angle of the endoscope in the first dimension. Specifically, a rotation of the first traction disk 2115 will control a winding/retraction or release of the first traction wire 21153 for the first dimension, thereby adjusting the extension distance of the first traction wire 21153.

Similar to the first traction disk, the second traction disk 2135 has a similar structure. The difference is that the second traction disk 2135 is arranged around the second rotating shaft 2133 at a position close to the second end. The second traction disk 2135 is configured to fix the second traction wire. A retraction and release of the second traction wire can be adjusted by rotating the second traction disk 2135, thereby adjusting the viewing angle of the endoscope in a second dimension. Specifically, rotation of the second traction disk 2135 will control a winding/retraction or release of the second traction wire for the second dimension, thereby adjusting the extension distance of the second traction wire. The traction wires are received within the catheter of the endoscope. For each traction wire, the two ends thereof are respectively positioned at the bending end and the traction disk end of the endoscope catheter. One end is connected to the corresponding traction disk, and the other end is fixed in the catheter. Generally speaking, in case one end of a traction wire is pulled by the corresponding traction disk, when the traction disk rotates, due to the flexibility of the catheter, the bending part at the distal end of the catheter will be driven by the traction wire to bend/flex, which in turn will drive the lens end of the endoscope to flex/rotate in a certain dimension (in an up-down direction, or in a left-right direction), allowing the lens of the endoscope to deflect to a certain angle and thus changing the viewing angle.

A spacer 2150 is provided in an axial gap between the first traction disk 2115 and the second traction disk 2135 to isolate the two disks.

The brake disc 2300 and the brake disc drive assembly 2500 will be introduced in the following. They will be introduced in a correlative manner since the two are closely related with each other. Please refer to FIG. 14 and FIG. 15.

The brake disc 2300 comprises: a brake disc body 2310, a brake disc dent 2330, an arc-shaped through hole 2350, and a positioning hole 2370. The brake disc driving assembly 2500 comprises: a driving member 2510 and a toggle lever 2530 (see FIG. 15). The driving member 2510 is provided close to the second end with respect to the brake disc driving assembly 2500 (close to a left side in the middle of FIG. 11), and the toggle lever 2530 is provided at the first end of the brake disc driving assembly 2500 (close to a right side in the middle of FIG. 11). A lower end of the toggle lever 2530 is fixedly connected to an outer circumferential surface of a driving member body 2515 in the driving member 2510. The toggle lever 2530 has a length that is set to be significantly larger than a diameter of the driving member 2510. An upper end of the toggle lever 2530 extends outwardly in the radial direction (towards the upper end in FIG. 11), and provides an easy-to-toggle operation surface. The structure of the driving member 2510 will be introduced in detail after the structure of the brake disc 2300 has been introduced later.

The handle 2700, whose name comes from its function as a handle of the endoscope, is equivalent to a case that provides a positioning foundation for other components in the present disclosure. The handle 2700 is generally designed as two covers engaged with each other that may be disassembled according to assembly/repair needs. For the endoscope locking device of the present disclosure, the relevant structure provided by the case mainly includes a fixing post 2710. In this embodiment, the handle 2700 In this embodiment, the handle 2700 has a hollow cylindrical shell made of plastic material. The fixing post 2710 is provided on an inner surface of the shell of the case 2700, and is configured to provide a mounting region for the brake disc 2300. In this embodiment, the fixing post 2710 is designed as a solid plastic cylinder, and the brake disc 2300 can be rotatably mounted/sleeved on the fixing post 2710 through its positioning hole 2370, so as to achieve a rotatable installation and positioning.

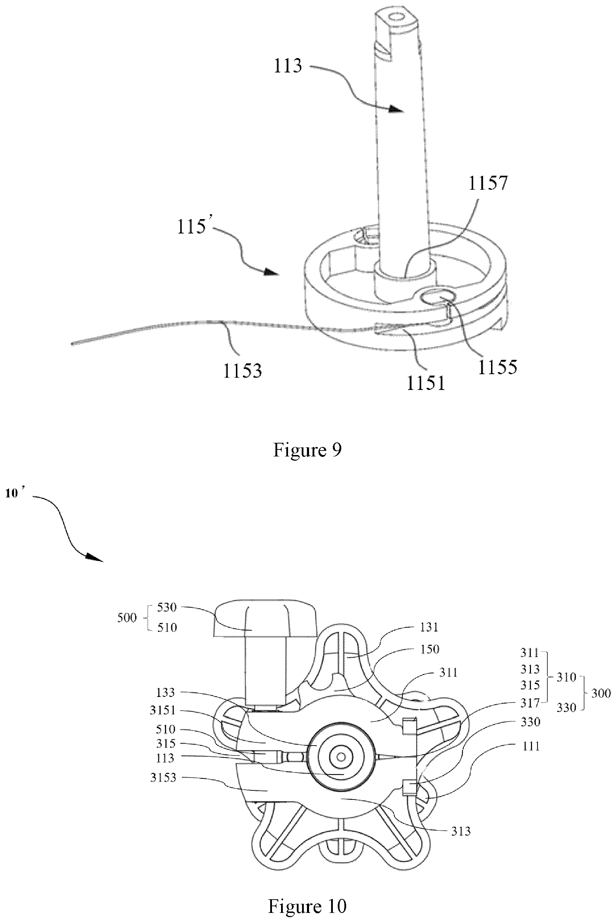

FIG. 14 shows a structural diagram of the brake disc 2300. The brake disc 2300 is described in detail below in conjunction with FIG. 14, and reference may also be made to FIG. 11 and FIG. 10.

As mentioned above, the brake disc 2300 comprises: a brake disc body 2310, a brake disc dent 2330, an arc-shaped through hole 2350, and a positioning hole 2370.

In this embodiment, the brake disc 2300 has a curved-panel structure as a whole. The brake disc 2300 is arranged on at least one side of the outer circumferential surface of the first rotating shaft 2113. The lower end of the brake disc body 2310 is provided with a brake disc dent 2330. The inner surface of the brake disc dent 2330 forms an engaging surface where the brake disc 2300 contacts the outer circumferential surface of the first rotating shaft 2130 and/or the second rotating shaft 2140. The inner surface of the brake disc dent 2330 is provided with a surface texture to increase friction, so as to improve an engagement with the outer circumferential surface of the first rotating shaft 2113 and/or the second rotating shaft 2133. The brake disc dent 2330 is coupled to the engagement surface of the first rotating shaft 2113 and the second rotating shaft 2133 through the O-ring 2170. The brake disc dent 2330 may apply a pressure on the O-ring 2170 via its surface texture when being driven by the brake disc body 2310, thus impedes a rotation of the first rotating shaft 2113 and the second rotating shaft 2133. A positioning hole 2370 is provided at one end of the curved panel of the brake disc body 2310. The positioning hole 2370 may be sleeved rotatably onto the fixing post 2710 on the handle 2700 , thereby realizing a fixed-axis swing of the brake disc body 2310 around the positioning hole 2370. An arc-shaped through hole 2350 is provided on the curved panel of the brake disc body 2310, so that the cantilever post 2519 may be inserted into the arc-shaped through hole 2350 and may slide within the arc-shaped through hole 2350. In order to obtain a synchronous engagement with the first rotating shaft 2113 and the second rotating shaft 2133, the brake disc body 2310 can be configured to include two curved panels that are parallelly arranged with respect to each other in fore-and-aft direction as shown in FIG. 14, namely a first brake disk body 2310-1 and a second brake disk body 2310-2.

FIG. 15 shows a structural diagram of a driving member 2510 as a component of the brake disc driving assembly 2500. The specific structure of the driving member 2510 is described in detail below in conjunction with FIG. 15, and reference may also be made to FIG. 11.

As shown in FIG. 15, the driving member 2510 comprises: driving member peripheral planes 2511, a driving member hollow hole 2513, a driving member body 2515, a cantilever 2517, and a cantilever post 2519.

The driving member body 2515 has a hollow tube configuration, and is provided at a lower end of the driving member 2510. A driving member hollow hole 2513 is provided in the driving member body 2515. The driving member body 2515 is sleeved on the outer circumferential surface of the second rotating shaft 2133 via the driving member hollow hole 2513. Two driving member peripheral planes 2511 are symmetrically provided on the outer circumferential surface of the driving member body 2515. A lower end of the driving member 2530 is fastened onto the driving member 2510 via the driving member peripheral plane 2511. By turning the toggle lever 2530, the driving member 2510 may be driven to rotate. A cantilever 2517 extending in the up-down direction is fixed at the front end (left side in FIG. 15) of the driving member body 2515, and a cantilever post 2519 is fixed at the upper front end (upper left side in FIG. 15) of the cantilever 2517. The cantilever post 2519 extends along the extension direction of the first rotating shaft 2113 and the second rotating shaft 2133, and is inserted into the arc-shaped through hole 2350 on the brake disc 2300. When the toggle lever 2530 is turned and swings, the driving member 2510 will be driven to rotate, thereby driving the cantilever post 2519 to slide in the arc-shaped through hole 2350.

During an operation of the endoscope, the toggle lever 2530 is turned to drive the driving member 2510 to rotate synchronously, so that the cantilever post 2519 slides in the arc-shaped through hole 2350. The movement of the cantilever post 2519 along the arc-shaped through hole 2350 in turn drives the brake disc 2300 to swing around the positioning hole 2370, so that the surface texture on the inner surface of the brake disc dent 2330 in the brake disc 2300 will provide a press/squeeze effect on the O-ring 2170. By this, a tightness of engagement between the brake disc 2300 and the outer circumferential surface of the first rotating shaft 2113 and the outer circumferential surface of the second rotating shaft 2133 may be adjusted, thereby providing the first rotating shaft 2113 and the second rotating shaft 2133 with a desired damping. Generally, via the above adjustment measure, the locking device 210 may have an unlocked state and a locked state, and can provide different degrees of damping in a intermediate position between the two states.

The above is just one implementation of the brake disc 2300. In fact, it is also possible to consider a clamping brake disc with multiple contact surfaces, so as to contact and engage with the outer circumferential surface of the first rotating shaft 2130 and/or the second rotating shaft 2140 from multiple sides to achieve braking effect. Those skilled in the art may carry out design under enlightenment of the embodiments disclosed above, in conjunction with technical knowledge in the art.

The working process of the locking device 210 will be introduced below in detail with reference to FIG. 12 and FIG. 13 mainly.

FIG. 12 shows a schematic diagram of the locking device 210 in FIG. 11, wherein the locking device is in an unlocked state. FIG. 13 a schematic diagram of the locking device 210 in FIG. 11, wherein the locking device is in a locked state. Both FIG. 12 and FIG. 13 are schematic structural diagrams of the locking device 210 in FIG. 11 after it is rotated counter-clockwise so as to be observed from the second end of the locking device 210, i.e., from the end on which the brake disc 2300 is provided. The working process of the locking device 210 will briefly introduced in the following with reference to FIG. 11 to FIG. 13, focusing on a process switching between the locked state and the unlocked state.

When the endoscope locking device 210 needs to be locked, the toggle lever 2530 is rotated along the handle 2700 clockwise by a certain angle (as shown in FIG. 12, from position A to position B). The toggle lever 2530 drives the driving member 2510 to rotate around the second rotating shaft 2133, which in turn drives the cantilever post 2519 on the driving member 2510 to slide in the arc-shaped through hole 2350, such that the brake disc 2300 is driven to rotate clockwise about the positioning hole 2370. The surface texture on the brake disc dent 2330 in the brake disc 2300 provides a press/squeeze effect on the O-ring 2170, such that at least one side of the outer circumferential surfaces of the first rotating shaft 2113 and the second rotating shaft 2133 is intimate contacted, engaged, and pressed. By this, a friction force acting on the first rotating shaft 2113 and the second rotating shaft 2133 is generated. The first rotating shaft 2113 and the second rotating shaft 2133 stop rotating under the friction force, and the first traction disk groove 21151 and the second traction disk groove 21351 stop retraction/release and winding of the traction wires configured for the left-right traction and up-down traction. The length of the traction wires for the left-right traction and up-down traction in the catheter in is fixed now, and the locking device enters into a locked state, that is, the angle and direction of the lens of the endoscope are locked. Referring to FIG. 12 and FIG. 13, the above process corresponds to a process changing from FIG. 12 to FIG. 13. It can be seen that the brake disc dent 2330 does not contact the first rotating shaft 2113 in FIG. 12, but will contact the first rotating shaft 2113 when it reaches the position of FIG. 13. The second rotating shaft 2133 is blocked at this viewing angle, but it actually develops in a similar way.

When the locking device 210 needs to be unlocked, the toggle lever 2530 is rotated along the handle 2700 counter-clockwise by a certain angle (as shown in FIG. 13, from position B to position A). The toggle lever 2530 drives the driving member 2510 to rotate around the second rotating shaft 2133, which in turn drives the cantilever post 2519 on the driving member 2510 to slide in the arc-shaped through hole 2350, such that the brake disc 2300 is driven to rotate counter-clockwise about the positioning hole 2370. This makes the surface texture on the brake disc dent 2330 in the brake disc 2300 get separate from the O-ring 2170, so that the friction force acting on the first rotating shaft 2113 and the second rotating shaft 2133 will gradually decrease until disappear. The locking device 210 gets released from the above-mentioned locked state and enters into the above-mentioned unlocked state. At this time, the first traction disk 2115 and the second traction disk 2135 may rotate readily as the operator operates the first rotating wheel 2111 and the second rotating wheel 2131. Traction wires correlated with the above-mentioned traction disks can be readily wound and retracted/released under the traction of the traction disks, so that the angle of the endoscope lens may be flexibly adjusted. Referring to FIG. 12 and FIG. 13, the above process corresponds to a process changing from FIG. 13 to FIG. 12. It can be seen that the surface texture on the brake disc dent 2330 contacts the first rotating shaft 2113 in FIG. 123, but does not contact the first rotating shaft 2113 when reaching the position of FIG. 13. The second rotating shaft 2133 is blocked at this viewing angle, but it actually develops in a similar way.

In a middle position between the above-mentioned locked position and unlocked position, the toggle lever 2530 may be operated/set to different positions between A and B, so that the locking device is under different damping, so as to adjust a tightness of the rotating wheels differently based on actual requirements. Through the locking device, the endoscope may be adjusted to a suitable state as needed. In the unlocked state, the lens angle of the endoscope can be freely adjusted via the rotating wheel. In the locked state, the endoscope is in a fixed state, and the lens angle will not change. In a damped state between the above two states, the present disclosure may provide the operator with the feel he needs to turn the wheel, thus making it easier to manipulate the device.

The following is a brief description of the principle of the above embodiments. The combination of the toggle lever 2530 and the driving member 2510 forms a lever mechanism. Since the toggle lever 2530 is relatively long and corresponds to a long arm in the lever mechanism, it may easily drives the driving member 2510 to rotate, which in turn will drive the brake disc 2300 to swing via the cantilever post 2519. On the contrary, it is difficult to drive the toggle lever 2530 via a swing of the brake disc 2300. Moreover, the cantilever post 2519 fits closely in the arc-shaped through hole 2350 of the brake disc 2300, so that there is a large friction force therebetween. Meanwhile, the toggle lever 2530 may be taken as a load that hinders a movement of the brake disc 2300. Therefore, a position of the brake disc 2300 is hardly to change due to reasons such as looseness. That is to say, the above-mentioned locking device has good locking characteristics. The toggle lever 2530, in the absence of an external force, may stay/remain in its place after it has been turned to an arbitrary position, and will not get loosened easily.

The first embodiment described above is a preferred embodiment. Obviously, there may also be other variations with respect to its basic principle. For example, the rotating wheel assembly 2100 may only be provided with the first rotating wheel assembly, that is, the locking device can only adjust the angle of the endoscope in one dimension. Of course, there are some other possible variations. For example, the first rotating wheel 2110 and the second rotating wheel 2120 mentioned hereinabove may be oppositely arranged, rather than being arranged at the same end as in this embodiment.

In the above embodiment, further, in the locking device for the endoscope, the brake disc and the brake disc driving assembly may also be divided into two groups to provide damping for the first rotating shaft and the second rotating shaft, respectively. Thus, the viewing angles of the endoscope in the first dimension and the second dimension can be controlled and adjusted separately. The second dimension and the first dimension described above are different dimensions for orientation.

Wherein, the brake disc 2300 can be divided, in vertical direction along the spacer 2150 in FIG. 11, into a first brake disc part and a second brake disc parts. Referring to the construction of the locking device in FIG. 11, a position and configuration of a first brake disc driving assembly may be similar to the brake disc driving assembly 2500 in FIG. 11 (right side in FIG. 11), and a second brake disc driving assembly may be mounted on the other side of the first brake disc driving assembly (left side in FIG. 11). The first brake disc part is arranged on a certain side of the outer circumferential surface of the first rotating shaft, and the second brake disc part is arranged on a certain side of the outer circumferential surface of the second rotating shaft. The first brake disc driving assembly may adjust a tightness of engagement between the first brake disc part and the outer circumferential surface of the first rotating shaft, so as to provide a desired damping to the first rotating shaft. The rotation of the first traction disk is used to adjust the first traction wire, thus to adjust the viewing angle of the endoscope in the first dimension. The second brake disc driving assembly may adjust a tightness of engagement between the second brake disc part and the outer circumferential surface of the second rotating shaft, so as to provide a desired damping to the second rotating shaft. The rotation of the second traction disk is used to adjust the second traction wire, thus to adjust the viewing angle of the endoscope in the second dimension.

According to the above preferred embodiment, it is possible to achieve an independent adjustment of the viewing angle of the endoscope in one dimension, while keeping the viewing angle in the other dimension unchanged/fixed.

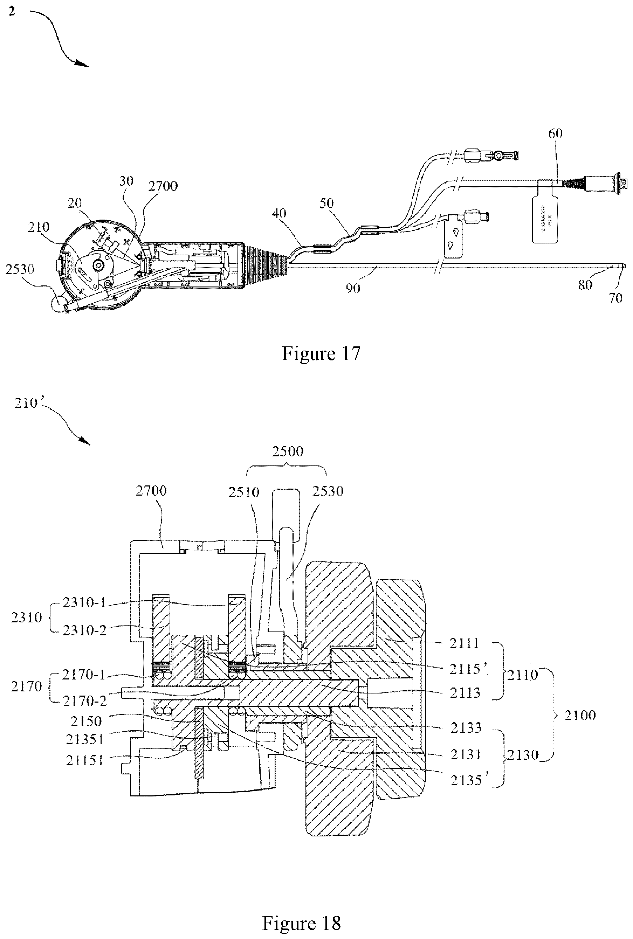

The fourth embodiment of the present disclosure provides an endoscope. Referring to FIG. 17, its structure and working process will be specifically described in conjunction with FIG. 11 to FIG. 16. In this embodiment, elements with the same functions as those in the above-mentioned third embodiment are named similarly as much as possible to facilitate understanding. It should be noted that although there are common innovation points between the third embodiment and the fourth embodiment, there are still significant differences between these two embodiments. Therefore, the description of this embodiment will be based on the nomenclature provided in this embodiment, there is no need for them to correspond exactly to the third embodiment constrainedly.

FIG. 17 illustrates a schematic diagram of the overall structure of the endoscope 2 provided in this embodiment.

The fourth embodiment of the present disclosure will be introduced in detail below in conjunction with FIG. 17.

FIG. 17 illustrates a schematic cross-sectional structural view of the endoscope 2 provide in this embodiment. Among which, the left side in FIG. 17 is referred to as a rear end of the endoscope, that is, an end at where the locking device 210 and the light source assembly 20 are arranged. The rear end of the endoscope is the end held by the operator during the actual operation. The right side in FIG. 17 is a front end of the endoscope structural device, that is, an end at where the lens 70 and the bending part 80 are arranged. The front end of the endoscope is configured to be motion controlled by the bending part during surgery, such that lesions of the relevant parts may be observed/examined. In this figure, the locking device 210 is in an unlocked state. In the following introduction, the left side in FIG. 17 is called "rear side", and the right side in FIG. 17 is called "front side".

As shown in FIG. 17, the endoscope 2 comprises: a locking device 210, a light source assembly 20, a traction wire 30, a suction tube 40, a flushing tube 50, a cable connector 60, a lens 70, a bending part 80, a catheter 90, a toggle lever 2530, and a handle 2700.

The light source assembly 20 and the locking device 210 are both accommodated in the handle 2700. The handle 2700 is configured to be held by the operator to manipulate the endoscope. The light source assembly 20 can provide an illumination light for the lens 70 during observation. The handle 2700 is provided on the rear end of the endoscope 2 (left side in FIG. 17), the bending part 80 and the lens 70 are provided at the front end of the endoscope 2 (right side in FIG. 17), and they are connected to each other through a catheter 90. During minimally invasive surgery, generally, the front end of the endoscope 2 is advanced to a surgical site along the patient's lumens in the unlocked state shown in FIG. 17. The traction wire 30, which connecting the lens 70 and the locking device 210, is wrapped within the catheter 90. The term "wrapped" here does not mean a tight wrapping, but means to provide a dedicated conduit for each traction wire, and the conduit has a suitable radial size. The catheter 90 itself is made of flexible material, so that it can adapt to curved lumens of the patient. The viewing angle of the lens 70 at the lesion site may be adjusted via the bending portion 80. The handle 2700 is held in the hands of a surgeon, and the surgeon can operate the toggle lever 2530 to control the locking device 210 outside the body as needed, thereby enabling observation of the lesion in the patient's body at different angles.

Wherein, via the locking device 210, the endoscope 2 may achieve any bending angle by the flexible traction wire 30. The locking device 210 may adjust an extension length of the traction wire 30, so as to adjust the angle of the lens 70 in a fixed position. Please refer to Embodiment 3 for the detailed process of adjusting the locking and non-locking working states of the locking device 210 via the toggle lever 2530, and it will not be described in detail here.

Although the endoscope 2 in this embodiment uses the locking device provided in the third embodiment, in fact, of course, the locking device provided in the first embodiment of the disclosure can also be used. In case said locking device is used, for a specific arrangement of the locking device, reference may be made to the description of the first embodiment, and will not be repeated here.

The front end of FIG. 17 (the upper right area in FIG. 17) also shows the suction tube 40, the flushing tube 50, and the cable connector 60 that cooperate with the lens 70. The suction tube 40 and the flushing tube 50 are used to remove visual obstructions in the observation area of the lens 70, so as to ensure a clear view of the operating field of the lens 70 and facilitate a direct observation of the lesions in the relevant parts. The cable connector 60 is used to gain access to required cables.

The fifth embodiment of the present disclosure provides a locking device. The purpose of providing this embodiment of the locking device is to extend the application of the locking device used in an endoscope provided in the third embodiment of the present disclosure to a wider range.

The structure and working process thereof will be described below in conjunction with FIG. 18. At the same time, reference can be made to FIG. 11 to FIG. 16. In this embodiment, elements with the same functions as those in the above-mentioned third embodiment are named similarly as much as possible to facilitate understanding. It should be noted that although there are common innovation points between the third embodiment and the fifth embodiment, there are still significant differences. Therefore, the description of this embodiment will be based on the nomenclature provided in this embodiment, there is no need for them to correspond exactly to the third embodiment constrainedly.

The locking device 210' is usually used in detection scenarios. Its working process will be described in conjunction with the third embodiment. The first traction disk 2115 and the second traction disk 2135 in the rotating wheel assembly 2100 are replaced with a first functional disc 2115' and a second functional disc 2135' which are used to realize various possible adjustment functions. It should be understood that the structures of the first functional disc 2115' and the second functional disc 2135' can be structurally adjusted according to the functions and application occasions of the locking device 210'. This embodiment is not specifically limited.

The locking device 210' comprises: a first rotating wheel 2111, a first rotating shaft 2113, a brake disc 2300, and a brake disc driving assembly 2500.

The first rotating wheel 2111 is coupled to a first end of the first rotating shaft 2113. A first functional disc 2115' is provided at a position of the first rotating shaft 2113 close to its second end. The brake disc 2300 is arranged on at least one side of the outer circumferential surface of the first rotating shaft 2113, and a tightness of engagement between the brake disc 2300 and the outer circumferential surface of the first rotating shaft 2113 may be adjusted by the brake disc driving assembly 2500. By this, a rotational damping on the first rotating shaft 2113 may fall within a first preset damping range, that is, a required damping may be provided on the first rotating shaft 2113 during its rotation.

Optionally, the locking device 210' further comprises: a second rotating wheel 2131 and a second rotating shaft 2133. The second rotating wheel 2131 is coupled to a first end of the second rotating shaft 2133. A second functional disc 2135' is provided at a position of the second rotating shaft 2133 close to its second end, and the second rotating shaft 2133 is coaxial with the first rotating shaft 2113. The brake disc 2300 is arranged on at least one side of the outer circumferential surface of the second rotating shaft 2133. During an adjustment of the engagement tightness between the brake disc 2300 and the outer circumferential surface of the first rotating shaft 2113, the tightness of engagement between the brake disc 2300 and the outer circumferential surface of the second rotating shaft 2133 may also adjusted synchronously, so that a rotational damping on the second rotating shaft 2133 may fall within the second preset damping range. By this, a required damping may be synchronously provided on the second rotating shaft 2133 during its rotation. It should be emphasized that FIG. 18 appears to have the same structure as FIG. 11 of the second embodiment. It is only used to exemplarily show that the components in the fifth embodiment have the same functions as the components in the second embodiment, and does not intend to limit their structures to be the same. It should be understood that the components in the fifth embodiment that have the same functions as those in the second embodiment may be the same or different in structure.

The locking device 210' provided by the fifth embodiment described above may also comprise other necessary structures, such as a detection device and a display device used to cooperate with the locking device. It should be understood that the working process of the locking device of this embodiment is similar to that of the third embodiment. A reference is made to the third embodiment, and will not be described in detail in this embodiment.

In the description of this specification, the description with reference to the terms "one embodiment", "some embodiments", "example", "specific example", or "some examples" etc. means that the specific features, structures, materials or characteristics described in conjunction with the embodiment or example are included in at least one embodiment or example of the present disclosure. In this specification, the schematic expressions of the above terms do not necessarily refer to the same embodiment or example. Moreover, the specific features, structures, materials or characteristics described may be combined into any of one or more embodiments or examples in a suitable manner. In addition, those skilled in the art may incorporate and combine the different embodiments or examples described in this specification and the features of the different embodiments or examples without contradiction.

Finally, it should be noted that the above implementations are only used to illustrate the technical solutions of the present disclosure, rather than to limit it. Although the present disclosure has been described in detail with reference to the aforementioned implementations, those ordinary skilled in the art should understand that they can still modify the technical solutions described in the aforementioned implementations, or replace some or all of the technical features therein with equivalents. However, these modifications or replacements do not cause the essence of the corresponding technical solutions to deviate from the scope of the technical solutions of the implementations of the present disclosure.

## Claims

1. A locking device for an endoscope, comprising a first rotating wheel, a first rotating shaft, a brake disc, and a brake disc driving assembly; wherein
the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; wherein a first traction wire may be adjusted via the rotation of the first traction disk, thereby adjusting a viewing angle of the endoscope in a first dimension; and
the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range.

2. The locking device for an endoscope according to claim 1, wherein the brake disc as a whole is configured as a frame structure clamping on the circumferential surface of the first rotating shaft or the circumferential surface of the first traction disk, wherein the frame structure forms a brake disc opening that may be opened/closed in an up-down direction;
the brake disc driving assembly has a fastener provided at least on one end of the brake disc opening, and a force applied to the brake disc may be adjusted by the fastener, such that the tightness of engagement between the brake disc and the outer circumferential surface of the first rotating shaft or the outer circumferential surface of the first traction disk may be adjusted by changing a clamping force exerted by the brake disc on the first rotating shaft or the first traction disk, so that the rotational damping on the first rotating shaft may fall within the first preset damping range.

3. The locking device for an endoscope according to claim 2, wherein a protrusion is provided at a base of the brake disc that is opposite to the brake disc opening, such that the brake disc may be fixed within a case of the locking device via the protrusionprotrusion.

4. The locking device for an endoscope according to claim 3, wherein a gap is provided at the base of the brake disc to increase a space for elastic deformation of the brake disc.

5. The locking device for an endoscope according to claim 3, wherein the protrusion is arranged between a first clamping post and a second clamping post provided on the case, so that the brake disc may be fixed within the case of the locking device.

6. The locking device for an endoscope according to claim 2, wherein the fastener comprises: a stud, and an upper nut and a lower nut that cooperate with the stud; the upper nut is arranged at an upper port of the brake disc opening, and the lower nut is arranged at a lower port of the brake disc opening;
wherein the upper nut and a first thread segment of the stud form a first thread pair, and the lower nut and a second thread segment of the stud form a second thread pair, with the first thread pair having a thread direction opposite to that of the second thread pair;
wherein the force applied to the brake disc may be adjusted by a rotation of the stud.

7. The locking device for an endoscope according to claim 2, wherein the fastener comprises a stud, and an internal thread cooperating with the stud is arranged at the opening of the frame structure; wherein an upper port of the opening cooperates with a first segment of the stud to form a third thread pair, and a lower port of the opening cooperates with a second segment of the stud to form a fourth thread pair, with the third thread pair having a thread direction opposite to that of the fourth thread pair;
wherein the force applied to the brake disc may be adjusted by a rotation of the stud.

8. The locking device for an endoscope according to claim 6 or claim 7, wherein the stud is provided with a gripping portion extending outside of a housing of the case, such that an operator of the locking device may use the gripping portion to rotate the stud.

9. The locking device for an endoscope according to claim 8, wherein the stud is provided with an end surface abutting against an outer surface of the housing of the case.

10. The locking device for an endoscope according to claim 2, further comprising a second rotating wheel and a second rotating shaft; wherein
the second rotating wheel is coupled to a first end of the second rotating shaft; wherein the second rotating shaft is coaxially arranged with respect to first rotating shaft, and is sleeved onto the outer circumferential surface of the first rotating shaft;
wherein a second traction disk is provided around the second rotating shaft at a position close to a second end of the second rotating shaft; wherein the second rotating shaft is controlled by the second rotating wheel to rotate, and in turn drives the second traction disk to rotate; wherein a second traction wire may be adjusted via the rotation of the second traction disk, thereby adjusting the viewing angle of the endoscope in a second dimension; the second dimension is a dimension for orientation that is different from the first dimension;
wherein the brake disc as a whole also clamps on an outer circumferential surface of the second rotating shaft or outer circumferential surface of the second traction disk;
when the brake disc exerts a clamping force on the first rotating shaft and the first traction disk, it also exerts a corresponding clamping force on the second rotating shaft and the second traction disk, such that a rotational damping on the second rotating shaft may fall within a second preset damping range.

11. The locking device for an endoscope according to claim 1, wherein the brake disc is arranged on one side of the outer circumferential surface of the first rotating shaft, and the tightness of engagement between the brake disc and the outer circumferential surface of the first rotating shaft may be adjusted via the brake disc driving assembly, so that the rotational damping on the first rotating shaft may fall within the first preset range.

12. The locking device for an endoscope according to claim 11, further comprising a second rotating wheel and a second rotating shaft; wherein
the second rotating wheel is coupled to a first end of the second rotating shaft; wherein the second rotating shaft is coaxially arranged with respect to first rotating shaft;
wherein a second traction disk is provided around the second rotating shaft at a position close to a second end of the second rotating shaft, and the brake disc is arranged on at least one side of an outer circumferential surface of the second rotating shaft; when the tightness of engagement between the brake disc and the outer circumferential surface of the first rotating shaft is adjusted, an engagement tightness between the brake disc and the outer circumferential surface of the second rotating shaft will be adjusted synchronously, so that a rotational damping on the second rotating shaft may fall within a second preset range;
wherein a second traction wire of the endoscope may be adjusted via a rotation of the second traction disk, thereby adjusting the viewing angle of the endoscope in a second dimension; the second dimension is a dimension for orientation that is different from the first dimension.

13. The locking device for an endoscope according to claim 12, wherein an O-ring is sleeved onto the outer circumferential surface of the first rotating shaft and/or the outer circumferential surface of the second rotating shaft at a location where the brake disc fits on the first rotating shaft and/or the second rotating shaft.

14. The locking device for an endoscope according to claim 12, wherein the brake disc is provided with a surface texture for increasing friction at its fitting surface engaging with the outer circumferential surface of the first rotating shaft and/or the outer circumferential surface of the second rotating shaft.

15. The locking device for an endoscope according to claim 12, wherein a positioning hole is provided at one end of a brake disc body, and is rotatably sleeved on a fixing post of the housing of the locking device; the tightness of engagement between the brake disc and the first rotating shaft and the second rotating shaft may be adjusted by adjusting a swing angle of the brake disc about the fixing post.

16. The locking device for an endoscope according to claim 15, wherein an arc-shaped through hole is provided on the brake disc body of the brake disc;
wherein the brake disc driving assembly comprises a driving member, with a body of driving member being coaxially arranged with respect to the first rotating shaft;
the driving member further has a cantilever that is connected to the body and extends radially towards one side; the cantilever is provided with a cantilever post that extends axially and is inserted into the arc-shaped through hole provided on the brake disc;
wherein a rotation of the driving member will drive the cantilever post to slide within the arc-shaped through hole, which in turn will drive the brake disc to swing about the fixing post, thereby adjusting an angle by which the brake disc swings about the fixing post.

17. The locking device for an endoscope according to claim 16, wherein the brake disc driving assembly further comprises a toggle lever having a length that is set to be significantly larger than a diameter of the body of the driving member;
wherein one end of the toggle lever is fixedly connected to the body of the driving member, and the other end of the toggle lever extends outwardly in the radial direction and provides an easy-to-toggle operation surface;
wherein a turning of the toggle lever will drive the driving member to rotate.

18. The locking device for an endoscope according to claim 12, wherein a spacer is provided at an axial gap formed between the first traction disk and the second traction disk.

19. The locking device for an endoscope according to claim 12, wherein the brake disc and the brake disc driving assembly are divided into two groups to provide damping for the first rotating shaft and the second rotating shaft, respectively.

20. An endoscope, comprising a first rotating wheel, a first rotating shaft, a brake disc, and a brake disc driving assembly; wherein
the first rotating wheel is coupled to a first end of the first rotating shaft; a first traction disk is provided around the first rotating shaft at a position close to a second end of the first rotating shaft; wherein the first rotating shaft is controlled by the first rotating wheel to rotate, and in turn drives the first traction disk to rotate; and
the brake disc is arranged on at least one side of an outer circumferential surface of the first rotating shaft or an outer circumferential surface of the first traction disk; a tightness of engagement between the brake disc and the outer circumferential surface of the first rotating shaft or the outer circumferential surface of the first traction disk may be adjusted by the brake disc driving assembly, such that a rotational damping on the first rotating shaft may fall within a first preset damping range;
wherein a first traction wire may be adjusted via the rotation of the first traction disk, thereby adjusting a viewing angle of the endoscope in a first dimension;
with such an adjustment and the damping provided by the brake disc, the lens of the endoscope can be driven to rotate into a required viewing angle in the first dimension and stay there as needed.

21. A locking device, comprising a first rotating wheel, a first rotating shaft, a brake disc, and a brake disc driving assembly; wherein
the first rotating wheel is coupled to a first end of the first rotating shaft; a first functional disc is provided around the first rotating shaft at a position close to a second end of the first rotating shaft; wherein the first rotating shaft is controlled by the first rotating wheel to rotate, and in turn drives the first functional disc to rotate; and
the brake disc is arranged on at least one side of an outer circumferential surface of the first rotating shaft or an outer circumferential surface of the first functional disc; a tightness of engagement between the brake disc and the outer circumferential surface of the first rotating shaft or the outer circumferential surface of the first functional disc may be adjusted by the brake disc driving assembly, such that a rotational damping on the first rotating shaft may fall within a first preset damping range.
